(19) Europäisches Patentamt
European Patent Office
Office européen des brevets

(11) **EP 4 501 335 A1**

(12) **EUROPEAN PATENT APPLICATION**
published in accordance with Art. 153(4) EPC

(43) Date of publication:
05.02.2025 Bulletin 2025/06

(21) Application number: 23781017.1

(22) Date of filing: 30.03.2023

(51) International Patent Classification (IPC):
*A61K 35/12* (2015.01)   *A61K 35/54* (2015.01)
*C07K 1/00* (2006.01)   *C07K 1/16* (2006.01)
*C12M 3/00* (2006.01)   *C12P 21/02* (2006.01)
*C12P 21/08* (2006.01)

(52) Cooperative Patent Classification (CPC):
A61K 35/12; A61K 35/54; C07K 1/00; C07K 1/16;
C07K 16/00; C12M 3/00; C12P 21/02

(86) International application number:
PCT/JP2023/013429

(87) International publication number:
WO 2023/191009 (05.10.2023 Gazette 2023/40)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 30.03.2022 JP 2022057528

(71) Applicant: FUJIFILM Corporation
Tokyo 106-8620 (JP)

(72) Inventor: KORI, Junichi
Ashigarakami-gun, Kanagawa 258-8577 (JP)

(74) Representative: Hoffmann Eitle
Patent- und Rechtsanwälte PartmbB
Arabellastraße 30
81925 München (DE)

(54) **PRODUCTION METHOD FOR ACTIVE INGREDIENT OF BIOLOGICAL MEDICINE, PRODUCTION SYSTEM FOR ACTIVE INGREDIENT OF BIOLOGICAL MEDICINE, AND ACTIVE INGREDIENT OF BIOLOGICAL MEDICINE**

(57)   Provided is a method for producing an active pharmaceutical ingredient of a biopharmaceutical, the method including: connecting, through a coupling line, a culture section where a culture step, in which cells are cultured in a culture liquid stored in a culture tank and the cells are removed from the culture liquid using a cell separation filter to obtain a culture supernatant liquid containing a product of the cells, is performed and a purification section where a purification step, in which the product is purified from the culture supernatant liquid to obtain an active pharmaceutical ingredient of a biopharmaceutical, is performed, the purification step including a purification step using a purification filter of a single pass tangential flow filtration method; providing, in the coupling line, a sterile filter having a pore diameter larger than a pore diameter of the cell separation filter; and filtering, with the sterile filter, the culture supernatant liquid flowing from the culture section to the purification section through the coupling line.

FIG. 1

**Description**

BACKGROUND OF THE INVENTION

1. Field of the Invention

The technology of the present disclosure relates to a method for producing an active pharmaceutical ingredient of a biopharmaceutical, a system for producing an active pharmaceutical ingredient of a biopharmaceutical, and an active pharmaceutical ingredient of a biopharmaceutical.

2. Description of the Related Art

A cell product such as an antibody is obtained by culturing a cell such as an antibody-producing cell in which an antibody gene is incorporated into Chinese Hamster Ovary cells (CHO cells), and an active pharmaceutical ingredient of a biopharmaceutical is obtained by purifying the product. In production of such an active pharmaceutical ingredient of a biopharmaceutical, in the related art, batch production in which a cell culture step and a cell product purification step are separately performed has been performed, but in recent years, continuous production has become mainstream instead of the batch production. The continuous production is a method in which a culture section where a culture step is performed, which includes a culture tank and the like, and a purification section where a purification step is performed, which includes a chromatography device and the like, are connected to each other physically, and the culture step and the purification step are continuously and consistently performed. According to the continuous production, quality and productivity of the active pharmaceutical ingredient can be significantly improved as compared with the batch production.

JP2020-033364A and JP2018-518161A disclose a system for producing an active pharmaceutical ingredient of a biopharmaceutical by the continuous production. In the production system disclosed in JP2020-033364A and JP2018-518161A, a sterile filter is provided between the culture section and the purification section. The sterile filter filters a culture supernatant liquid flowing from the culture section to the purification section. The culture supernatant liquid is obtained by removing cells from a culture liquid of cells by a cell separation filter provided in the culture section, and mainly contains a product. With the sterile filter, it is possible to prevent bacteria from invading from the purification section to the culture section, and it is possible to reduce the concern of contamination of the culture supernatant liquid.

In JP2020-033364A and JP2018-518161A, it is exemplified that a pore diameter of the cell separation filter located upstream is 0.2 $\mu$m. In addition, in JP2020-033364A and JP2018-518161A, it is exemplified that a pore diameter of a sterile filter located downstream is 0.2 $\mu$m which is the same as that of the cell separation filter, or a value larger than that of the cell separation filter. In addition, in JP2020-033364A and JP2018-518161A, a purification step using a chromatography device is mainly performed.

SUMMARY OF THE INVENTION

In a case where the pore diameter of the sterile filter is the same as that of the cell separation filter or larger than that of the cell separation filter, as in JP2020-033364A and JP2018-518161A, a load on the sterile filter located downstream is reduced. Therefore, it is less likely that a malfunction such as clogging in the sterile filter occurs, and maintainability is improved.

However, in a case where the purification step is mainly performed using a chromatography device, the maintainability is deteriorated due to the occurrence of malfunction.

One embodiment according to the technology of the present disclosure provides a method for producing an active pharmaceutical ingredient of a biopharmaceutical, a system for producing an active pharmaceutical ingredient of a biopharmaceutical, and an active pharmaceutical ingredient of a biopharmaceutical, with which it is possible to suppress a decrease in maintainability.

A method for producing an active pharmaceutical ingredient of a biopharmaceutical according to the present disclosure includes connecting, through a coupling line, a culture section where a culture step, in which cells are cultured in a culture liquid stored in a culture tank and the cells are removed from the culture liquid using a cell separation filter to obtain a culture supernatant liquid containing a product of the cells, is performed and a purification section where a purification step, in which the product is purified from the culture supernatant liquid to obtain an active pharmaceutical ingredient of a biopharmaceutical, is performed, the purification step including a purification step using a purification filter of a single pass tangential flow filtration method; providing, in the coupling line, a sterile filter having a pore diameter larger than a pore diameter of the cell separation filter; and filtering, with the sterile filter, the culture supernatant liquid flowing from the culture section to the purification section through the coupling line.

It is preferable that a subsequent step of the purification step using the purification filter of the single pass tangential flow filtration method is a purification step using a chromatography device.

It is preferable that the chromatography device is a single column.

It is preferable that a sterile filter connected in parallel to the coupling line is used in addition to the sterile filter provided in the coupling line.

It is preferable that, in a case where a maximum accommodation capacity of the culture tank is denoted by MCC and a filtration area of the sterile filter is denoted by FA, $0.001 \text{ m}^2/\text{L} \le (\text{FA}/\text{MCC}) \le 0.01 \text{m}^2/\text{L}$.

It is preferable that the pore diameter of the sterile filter is larger than a pore diameter of the purification filter of the single pass tangential flow filtration method, and is 0.1 μm or more and 1.0 μm or less.

It is preferable that the sterile filter has a structure in which a flat filter paper is folded.

It is preferable that the sterile filter has a structure in which hollow fibers are bundled together.

It is preferable that a material of the sterile filter is polyethersulfone.

It is preferable that the culture supernatant liquid is sent from the culture section toward the purification section by a delivery pump provided upstream of the sterile filter in the coupling line.

It is preferable that the culture supernatant liquid is temporarily stored in an intermediate container provided downstream of the sterile filter in the coupling line.

It is preferable that a flow rate of the culture supernatant liquid to the sterile filter is constant.

It is preferable that, in a case where an integrated flow rate of the culture supernatant liquid to the sterile filter has reached a set amount set to be $1,000 \text{ L/m}^2$ to $10,000 \text{ L/m}^2$, the sterile filter is exchanged.

It is preferable that a flow rate of the culture supernatant liquid flowing into the purification filter is changed by 50% or more at a set interval set to be 2 minutes to 30 minutes.

It is preferable that the culture supernatant liquid is allowed to flow into a sampling container connected downstream of the sterile filter in the coupling line.

It is preferable that the culture supernatant liquid is allowed to flow into the sampling container using a supply pump provided in a branch line connecting the coupling line and the sampling container.

It is preferable that the product is a protein.

It is preferable that the cell is a cell derived from Chinese Hamster Ovary cell.

A system for producing an active pharmaceutical ingredient of a biopharmaceutical according to the present disclosure includes a culture section where a culture step, in which cells are cultured in a culture liquid stored in a culture tank and the cells are removed from the culture liquid using a cell separation filter to obtain a culture supernatant liquid containing a product of the cells, is performed; a purification section where a purification step, in which the product is purified from the culture supernatant liquid to obtain an active pharmaceutical ingredient of a biopharmaceutical, is performed, the purification step including a purification step using a purification filter of a single pass tangential flow filtration method; a coupling line coupling the culture section and the purification section; and a sterile filter which is provided in the coupling line, has a pore diameter larger than a pore diameter of the cell separation filter, and filters the culture supernatant liquid flowing from the culture section to the purification section through the coupling line.

An active pharmaceutical ingredient of a biopharmaceutical according to the present disclosure is produced by the method for producing an active pharmaceutical ingredient of a biopharmaceutical described above.

According to the technology of the present disclosure, it is possible to provide a method for producing an active pharmaceutical ingredient of a biopharmaceutical, a system for producing an active pharmaceutical ingredient of a biopharmaceutical, and an active pharmaceutical ingredient of a biopharmaceutical, with which it is possible to suppress a decrease in maintainability.

BRIEF DESCRIPTION OF THE DRAWINGS

Fig. 1 is a diagram showing a system for producing an active pharmaceutical ingredient of a biopharmaceutical.
Fig. 2 is a diagram showing a periphery of a sterile filter.
Fig. 3 is a diagram showing a structure of a sterile filter and an intermediate container.
Fig. 4 is a block diagram of a computer constituting a control device.
Fig. 5 is a block diagram of a CPU of the computer constituting the control device.
Fig. 6 is a diagram showing a change in time of a liquid amount of a culture supernatant liquid in an intermediate container, an operation timing of a first supply pump, and an operation timing of a purification section.
Fig. 7 is a flowchart showing a processing procedure of the control device.
Fig. 8 is a diagram showing another example of a method of measuring a liquid amount of a culture supernatant liquid.
Fig. 9 is a diagram showing a time change of a flow rate of a culture supernatant liquid flowing into a purification filter of a single pass tangential flow filtration method.
Fig. 10 is a diagram showing a change in time of a liquid amount of a culture supernatant liquid in the intermediate container in the example shown in Fig. 9.

Fig. 11 is a diagram showing a second embodiment in which a plurality of sterile filters are used.

Fig. 12 is a diagram showing an intermediate section control unit of the second embodiment.

Fig. 13 is a diagram showing processing of the intermediate section control unit of the second embodiment, in which a left side of Fig. 13 shows a case in which an integrated flow rate of the culture supernatant liquid to a sterile filter of a coupling path does not reach a set amount, and a right side of Fig. 13 shows a case in which the integrated flow rate of the culture supernatant liquid to the sterile filter of the coupling path reaches the set amount.

Fig. 14 is a diagram showing another example of the sterile filter.

DESCRIPTION OF THE PREFERRED EMBODIMENTS

[First embodiment]

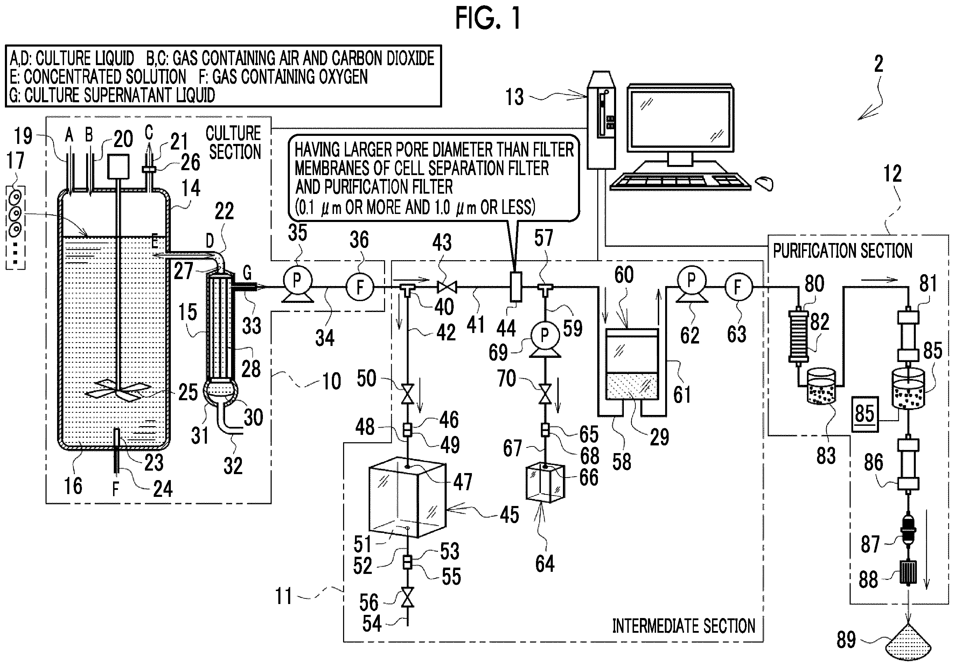

As an example shown in Fig. 1, a system 2 for producing an active pharmaceutical ingredient of a biopharmaceutical according to the technology of the present disclosure, which is used for performing a method for producing an active pharmaceutical ingredient of a biopharmaceutical, includes a culture section 10 where a culture step is performed, an intermediate section 11, a purification section 12 where a purification step is performed, and a control device 13. The culture section 10 includes a culture tank 14 and a cell separation filter 15. A culture liquid (also referred to as a culture solution) 16 is stored in the culture tank 14. Antibody-producing cells 17 are seeded in the culture tank 14, and are cultured in the culture liquid 16.

The antibody-producing cells 17 are, for example, cells established by incorporating an antibody gene into a cell such as Chinese Hamster Ovary cell. That is, the antibody-producing cells 17 are an example of "cell derived from Chinese Hamster Ovary cell" according to the technology of the present disclosure. The antibody-producing cells 17 produce an immunoglobulin, that is, an antibody 18 as a product in the process of culturing. Therefore, not only the antibody-producing cells 17 but also the antibody 18 is present in the culture liquid. The antibody 18 is, for example, a monoclonal antibody, and is an active ingredient of a biopharmaceutical. The antibody 18 is an example of "protein" according to the technology of the present disclosure.

The culture tank 14 is provided with a culture liquid supply path 19, a gas supply path 20, an exhaust path 21, a culture liquid delivery and recovery path 22, a sparger 23, a gas supply path 24, a stirring blade 25, and the like. The culture liquid supply path 19 is a flow passage for continuously supplying a fresh culture liquid 16 into the culture tank 14, as indicated by an arrow A. That is, the culture section 10 performs perfusion culture. The gas supply path 20 is a flow passage for supplying a gas containing air and carbon dioxide from the upper part, as indicated by an arrow B. As indicated by an arrow C, the exhaust path 21 is a flow passage for exhausting the gas supplied from the gas supply path 20 to the outside of the culture tank 14. An exhaust filter 26 is provided in the exhaust path 21.

The culture liquid delivery and recovery path 22 is connected to an inlet and outlet 27 of the cell separation filter 15. The culture liquid delivery and recovery path 22 is a flow passage for delivering the culture liquid 16 in the culture tank 14 to the cell separation filter 15, as indicated by an arrow D. In addition, the culture liquid delivery and recovery path 22 is a flow passage for returning the culture liquid 16 (concentrated solution) from the cell separation filter 15 to the culture tank 14, as indicated by an arrow E. Although not shown, a pump which supplies the culture liquid 16 into the culture tank 14 is provided in the culture liquid supply path 19.

The sparger 23 is disposed at the bottom of the culture tank 14. As indicated by an arrow F, the sparger 23 releases gas containing oxygen, which is supplied from the gas supply path 24, to the culture tank 14. The oxygen released from the sparger 23 is dissolved in the culture liquid 16, and supports production activity of the antibody 18 of the antibody-producing cells 17. The stirring blade 25 is rotated at a predetermined rotation speed by a motor or the like to stir the culture liquid 16 in the culture tank 14. As a result, homogeneity of the culture liquid 16 in the culture tank 14 is maintained. In addition to these members, the culture tank 14 is provided with a flow passage for a cell breeding treatment of intentionally taking out a part of the culture liquid 16, for example. The stirring blade 25 may have a plurality of blades as shown in the drawing or may have one disk-shaped blade, and the shape thereof is not particularly limited. In addition, two or more stirring blades 25 may be arranged in the culture tank 14.

The cell separation filter 15 has a filter membrane 28 inside. The filter membrane 28 captures the antibody-producing cells 17 and transmits the antibody 18. With the cell separation filter 15, a culture supernatant liquid 29 is obtained by, for example, Tangential Flow Filtration (TFF) method in which the antibody-producing cells 17 are removed from the culture liquid 16 by the filter membrane 28. A pore diameter of the filter membrane 28 is not particularly limited as long as it is a size capable of removing cells such as the antibody-producing cells 17 from the culture liquid 16, and it may be 0.01 $\mu$m or more and 0.5 $\mu$m or less, preferably 0.1 $\mu$m or more and 0.4 $\mu$m or less and particularly preferably 0.15 $\mu$m or more and 0.3 $\mu$m or less. The pore diameter of the filter membrane 28 is, for example, 0.2 $\mu$m. The "pore diameter" used herein means an average pore diameter. The average pore diameter can be measured by mercury porosimetry.

More specifically, the cell separation filter 15 has a diaphragm pump 31 having an elastic film 30 inside, and a degassing and gas suppling path 32. Air on the lower side of the elastic film 30 is degassed from the degassing and gas suppling path 32, and the elastic film 30 is elastically deformed to be attached to the lower end of the diaphragm pump 31, whereby the culture liquid 16 in the culture tank 14 flows into the cell separation filter 15 through the culture liquid delivery and recovery path 22. In addition, the culture liquid 16 (concentrated solution) which cannot pass through the filter membrane 28 is returned to the culture tank 14 through the culture liquid delivery and recovery path 22, by supplying air from the degassing and gas suppling path 32 to the lower side of the elastic film 30 and elastically deforming the elastic film 30 to be attached to the upper side of the diaphragm pump 31.

The culture supernatant liquid 29 flows out from an outlet 33 of the cell separation filter 15, as indicated by an arrow G. The culture supernatant liquid 29 mainly contains the antibody 18. The culture supernatant liquid 29 contains, in addition to the antibody 18, contaminants such as a cell-derived protein, cell-derived Deoxyribonucleic Acid (DNA), and an aggregate of the antibody 18, a virus, and the like.

A coupling path 34 is connected to an outlet 33 of the cell separation filter 15. The coupling path 34 is provided with a delivery pump 35 and a flowmeter 36. The delivery pump 35 sends out the culture supernatant liquid 29 flowed out from the outlet 33 downstream of the intermediate section 11 and the purification section 12. The flowmeter 36 measures a flow rate of the culture supernatant liquid 29 flowing through the coupling path 34.

In the intermediate section 11, the coupling path 34 is sterilely connected to a coupling path 41 and a branch path 42 through a three-way joint 40. The coupling path 41 is provided with a pinch valve 43 and a sterile filter 44. The pinch valve 43 and the sterile filter 44 are provided downstream of the three-way joint 40, which is the connecting portion with the branch path 42, in the coupling path 41.

The pinch valve 43 is a valve for switching a flow passage of the culture supernatant liquid 29 from the coupling path 41 to the branch path 42. The pinch valve 43 is, for example, a valve which closes the coupling path 41 by flattening the tube constituting the coupling path 41 from the outside with a valve body by the action of a coil and a movable iron core. The pinch valve 43 flattens the tube constituting the coupling path 41 to block the flow of the culture supernatant liquid 29 from the culture section 10 into the purification section 12 through the coupling path 41. In this way, the pinch valve 43 is a method of pinching the tube from the outside, and the flow passage can be switched without contacting the culture supernatant liquid 29. Since other pinch valves 50 and 70 described later also have the same configuration and the same action as the pinch valve 43, except that the tube flattening with the valve body is different, the detailed description thereof will be omitted. In addition, since a pinch valve 56 described later has the same configuration and the same operation as the pinch valve 43, except that the tube flattening with the valve body is different and the pinch valve 56 is manually operated, the detailed description thereof will be omitted.

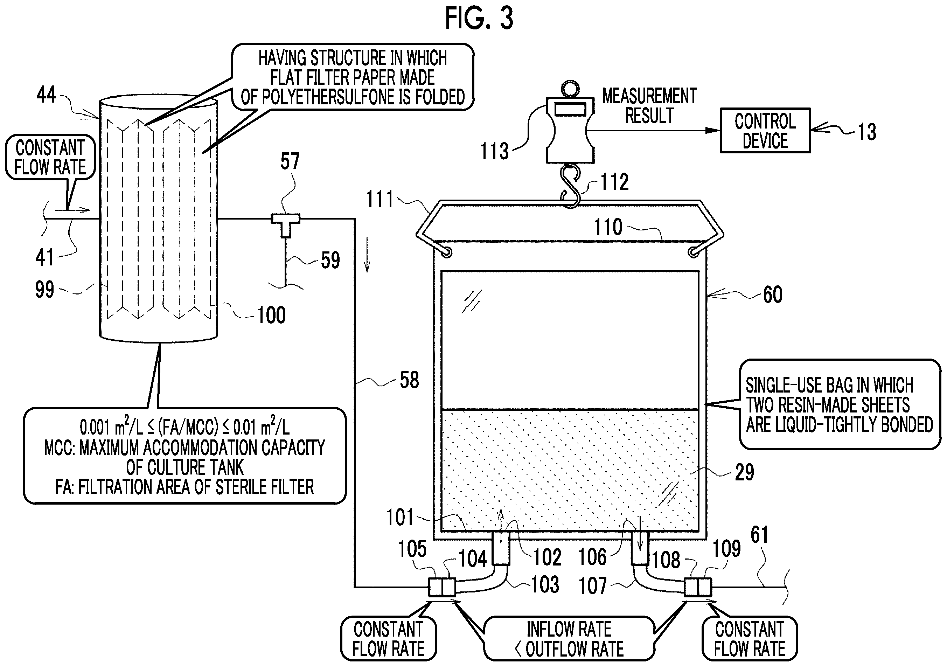

The sterile filter 44 filters the culture supernatant liquid 29 flowing into the purification section 12. The sterile filter 44 incorporates a filter membrane 99 and a filter membrane 100 (see Fig. 3 for both) having a pore diameter larger than the filter membrane 28 of the cell separation filter 15 and a purification filter 82 of a single pass tangential flow filtration (hereinafter, abbreviated as SPTFF) device 80 described later. The filter membrane 100 has a pore diameter smaller than that of the filter membrane 99. The pore diameter of the filter membranes 99 and 100 satisfies the following conditions, respectively.

Pore diameter of filter membrane 99: 0.4 $\mu$m or more and 2.0 $\mu$m or less, preferably 0.4 $\mu$m or more and 1.0 $\mu$m or less
Pore diameter of filter membrane 100: 0.1 $\mu$m or more and 1.0 $\mu$m or less, preferably 0.1 $\mu$m or more and 0.3 $\mu$m or less

A tank 45 is connected to the branch path 42. The tank 45 temporarily stores the culture supernatant liquid 29 from the culture section 10 in a case where a malfunction occurs in the purification section 12. The tank 45 is formed of, for example, a resin film such as polyolefin (PO) and ethylene-vinyl acetate (EVA) copolymer in a rectangular shape, and has flexibility. In addition, the tank 45 is made of a single-use material which can be used only once.

The tank 45 has an accommodation capacity of 0.2 times or more and 10 times or less the amount of the culture liquid 16 in the culture tank 14. Therefore, for example, in a case where the amount of the culture liquid 16 is 50 L, the accommodation capacity of the tank 45 is 10 L to 500 L. In addition, for example, in a case where the amount of the culture liquid 16 is 5,000 L, the accommodation capacity of the tank 45 is 1,000 L to 50,000 L.

A sterile connector 46 is provided at a distal end of the branch path 42. In addition, a sterile connector 49 is also provided at a distal end of a flow-in tube 48 connected to an inlet port 47 of the tank 45 for the culture supernatant liquid 29. Through the sterile connectors 46 and 49, the branch path 42 is sterilely connected to the flow-in tube 48, and thus sterilely connected to the tank 45. The flow passage of the culture supernatant liquid 29 from the culture section 10 to the tank 45 is all sterilely connected by the sterile connectors 46 and 49, the three-way joint 40, and the like. As the sterile connector 46 and the like (including sterile connectors 53, 55, 65, 68, and the like described later), for example, ReadyMate™ Disposable Aseptic Connector (DAC) manufactured by Global Life Science Technologies Japan, Inc. can be used.

The branch path 42 is provided with a pinch valve 50. The pinch valve 50 is a valve for allowing the culture supernatant liquid 29 to flow into the tank 45 through the branch path 42.

A liquid level sensor (not shown) is provided in the tank 45. In a case where the liquid level sensor detects that the culture supernatant liquid 29 has reached a predetermined water level, a signal indicating that the tank 45 is filled with the culture supernatant liquid 29 is transmitted to the control device 13.

A discharge port 51 is provided at the bottom of the tank 45. A discharge tube 52 is connected to the discharge port 51. A sterile connector 53 is provided at a distal end of the discharge tube 52. A waste liquid path 54 is connected to the discharge tube 52. A sterile connector 55 is provided at a distal end of the waste liquid path 54. Through the sterile connectors 53 and 55, the waste liquid path 54 is sterilely connected to the discharge tube 52, and thus sterilely connected to the tank 45.

A manual pinch valve 56 is provided in the waste liquid path 54. In a case where the pinch valve 56 is opened, the culture supernatant liquid 29 temporarily stored in the tank 45 is discarded as waste liquid to a waste liquid tank (not shown) through the discharge port 51, the discharge tube 52, and the waste liquid path 54. The reason why the culture supernatant liquid 29 from the culture section 10 is once stored in the tank 45 and then discarded into the waste liquid path 54, without being directly discarded into the waste liquid path 54, is to prevent contamination from the waste liquid path 54. Although not shown, a sterile filter may be installed between the waste liquid path 54 and the waste liquid tank.

The tank 45 is separated from the branch path 42 by removing the sterile connector 49 from the sterile connector 46 after the pinch valve 56 is manually opened and the culture supernatant liquid 29 is discarded. A sterile connector 49 of a new empty tank 45 is attached to the sterile connector 46. As a result, the used tank 45 is exchanged for the new empty tank 45.

A configuration may be adopted in which a further branch path is connected to the upstream side of the branch path 42 with respect to the pinch valve 50, and a plurality of tanks 45 can be connected. In addition, the tank 45 and the coupling path 41 may be connected to each other, and the culture supernatant liquid 29 temporarily stored in the tank 45 may be returned to the coupling path 41 and flow into the purification section 12.

Downstream of the sterile filter 44, the coupling path 41 is sterilely connected to a coupling path 58 and a branch path 59 through a three-way joint 57. An intermediate container 60 is connected to the coupling path 58. The intermediate container 60 temporarily stores the culture supernatant liquid 29 in front of the purification section 12. A coupling path 61 is connected to the intermediate container 60. The coupling path 61 is connected to the purification section 12. The coupling path 61 is provided with a first supply pump 62 and a flowmeter 63. The first supply pump 62 supplies the culture supernatant liquid 29 in the intermediate container 60 to the purification section 12. The flowmeter 63 measures a flow rate of the culture supernatant liquid 29 flowing through the coupling path 61, that is, a flow rate of the culture supernatant liquid 29 flowing into the purification section 12. The coupling paths 34, 41, 58, and 61 are an example of "coupling line" according to the technology of the present disclosure.

A sampling container 64 is connected to the branch path 59. The branch path 59 is an example of "branch line" according to the technology of the present disclosure. The sampling container 64 is a container for sampling a small amount of the culture supernatant liquid 29 for sampling the concentration of the antibody 18, and the like in the culture supernatant liquid 29. Same as the tank 45, the sampling container 64 is formed of a resin film such as polyolefin and ethylene-vinyl acetate copolymer in a rectangular shape, and has flexibility. In addition, the sampling container 64 is made of a single-use material which can be used only once, same as the tank 45. However, the sampling container 64 has a smaller accommodation capacity than the tank 45.

A sterile connector 65 is provided at a distal end of the branch path 59. In addition, a sterile connector 68 is also provided at a distal end of a flow-in tube 67 connected to an inlet port 66 of the sampling container 64 for the culture supernatant liquid 29. Through the sterile connectors 65 and 68, the branch path 59 is sterilely connected to the flow-in tube 67, and thus sterilely connected to the sampling container 64.

The branch path 59 is provided with a second supply pump 69 and a pinch valve 70. The second supply pump 69 introduces the culture supernatant liquid 29 flowing through the coupling path 41 into the branch path 59. The pinch valve 70 is a valve for allowing the culture supernatant liquid 29 led into the branch path 59 by the second supply pump 69 to flow into the sampling container 64. The second supply pump 69 is an example of "supply pump" according to the technology of the present disclosure.

The sampling container 64 is separated from the branch path 59 after a sufficient amount of the culture supernatant liquid 29 is flowed thereinto for sampling, by removing the sterile connector 68 from the sterile connector 65. The separated sampling container 64 is placed in a sampling device such as an antibody concentration determination device. A sterile connector 68 of a new sampling container 64 is attached to the sterile connector 65. As a result, the used sampling container 64 is exchanged for the new sampling container 64. Same as the tank 45, a configuration may be adopted in which a further branch path is connected between the second supply pump 69 and the pinch valve 70 of the branch path 59, and a plurality of sampling containers 64 can be connected.

The purification section 12 includes the SPTFF device 80, an immunoaffinity chromatography device 81, and the like, and continuously purifies the culture supernatant liquid 29 by these devices 80, 81, and the like. The culture supernatant liquid 29 flows into the SPTFF device 80 through the coupling path 61. The SPTFF device 80 includes a purification filter 82 of the SPTFF method. The purification filter 82 is composed of a plurality of ultrafiltration (UF) filter membranes connected in series. With the purification filter 82, the antibody 18 can be sufficiently concentrated only by passing the culture supernatant liquid 29 once without requiring the loop of the culture supernatant liquid 29. The SPTFF device 80 generates a first purified liquid 83 by extracting the antibody 18 from the culture supernatant liquid 29 using the purification filter 82.

The first purified liquid 83 extracted using the purification filter 82 flows into the immunaffinity chromatography device 81. The immunaffinity chromatography device 81 extracts the antibody 18 from the culture supernatant liquid 29 using a column in which a ligand such as protein A and protein G, having an affinity for the antibody 18, is immobilized on a carrier, thereby generating a second purified liquid 84. That is, in the purification section 12, the first step is a purification step using the purification filter 82 of the SPTFF method, and the next step is a purification step using the immunaffinity chromatography device 81 with a single column.

The second purified liquid 84 is subjected to a treatment 85 for inactivating the virus (hereinafter, referred to as a virus inactivating treatment). The second purified liquid 84 subjected to the virus inactivating treatment 85 is further purified by an ion affinity chromatography device 86 with a column in which a cation exchanger is used as a stationary phase and/or with a column in which an anion exchanger is used as a stationary phase, and then passed through a filter 87 to remove the virus. Thereafter, the second purified liquid 84 is subjected to a concentration and filtration treatment by an ultrafiltration and diafiltration (DF) through a filter 88. As a result, an active pharmaceutical ingredient 89 of a biopharmaceutical is obtained. Accordingly, by sequentially performing the component separation treatments by the SPTFF device 80, the immunaffinity chromatography device 81, and the like, the contaminants and virus are stepwise removed from the culture supernatant liquid 29, and the purity of the antibody 18 is stepwise increased. In this way, the purification section 12 is a site where the concentration of the purified target such as the antibody 18 and the removal of the impurities other than the purified target, such as the virus, are intentionally performed by making full use of the SPTFF device 80 and the like.

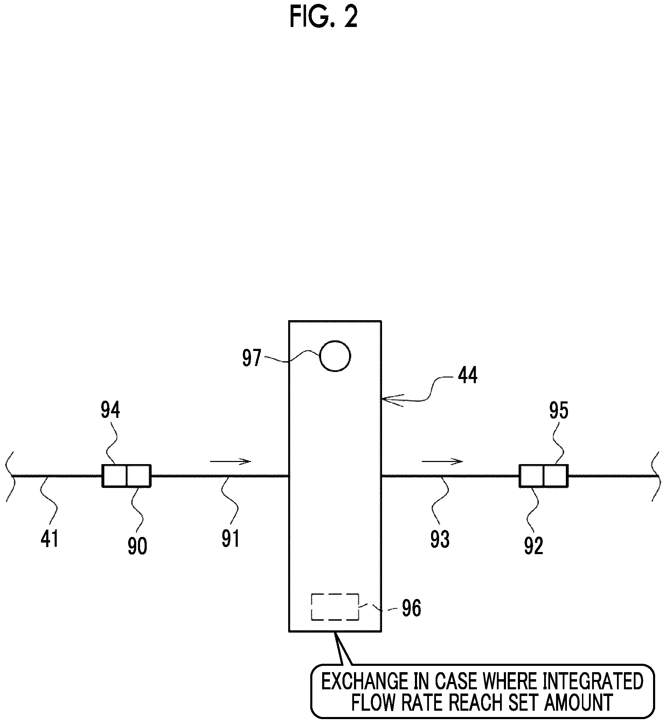

As an example shown in Fig. 2, the sterile filter 44 includes a flow-in tube 91 having a sterile connector 90, and a flow-out tube 93 having a sterile connector 92. On the other hand, sterile connectors 94 and 95 are provided on the upstream side and the downstream side of the coupling path 41. The sterile connector 90 is connected to the sterile connector 94, and the sterile connector 92 is connected to the sterile connector 95, whereby the sterile filter 44 is attached to the coupling path 41. The sterile filter 44 is sterilely connected to the coupling path 41 through the sterile connectors 90, 92, 94, and 95.

The sterile filter 44 is provided with an integrated flowmeter 96 which measures an integrated flow rate of the culture supernatant liquid 29. In addition, the sterile filter 44 is provided with an indicator 97 which reports that an integrated flow rate of the culture supernatant liquid 29 measured by the integrated flowmeter 96 has reached a set amount. The indicator 97 is, for example, a rotating disk having a white background and a red background, and displays the white background in a case where the integrated flow rate of the culture supernatant liquid 29 does not reach the set amount, and displays the red background in a case where the integrated flow rate of the culture supernatant liquid 29 reaches the set amount. Alternatively, the indicator 97 is a light emitting element such as Light-Emitting Diode (LED). The set amount is set in a range of 1,000 L/m$^2$ to 10,000 L/m$^2$. The integrated flowmeter 96 may not be incorporated in the sterile filter 44, and may be provided upstream of the sterile filter 44 separately from the sterile filter 44.

In a case where the integrated flow rate of the culture supernatant liquid 29 has reached the set amount, the sterile filter 44 is separated from the coupling path 41 by removing the sterile connectors 90 and 92 from the sterile connectors 94 and 95. Sterile connectors 90 and 92 of a new sterile filter 44 are attached to the sterile connectors 94 and 95. As a result, the sterile filter 44 in which the integrated flow rate of the culture supernatant liquid 29 has reached the set amount is exchanged for the new sterile filter 44.

As an example shown in Fig. 3, the flow rate of the culture supernatant liquid 29 flowing into the sterile filter 44 by the delivery pump 35 is constant. The "constant" refers to a certain in the sense of including an error generally allowed in the technical field to which the technology of the present disclosure belongs, that is, an error to the extent that it does not contradict the gist of the technology of the present disclosure, in addition to the complete certainty. In the technology of the present disclosure, the "constant" means that the flow rate of the culture supernatant liquid 29 flowing into the sterile filter 44 need only be within ±20% of a target value, and preferably within ±10% of the target value.

The sterile filter 44 includes the filter membranes 99 and 100. The filter membranes 99 and 100 have a structure in which a flat filter paper made of polyethersulfone is folded. The sterile filter 44 having such filter membranes 99 and 100 is referred to as a flat membrane filter.

In a case where the maximum accommodation capacity of the culture tank 14 is denoted by MCC and a filtration area of the filter membranes 99 and 100 of the sterile filter 44 is denoted by FA, a ratio FA/MCC of the maximum accommodation capacity MCC of the culture tank 14 to the filtration area FA of the filter membranes 99 and 100 may be in a range of Expression (1), preferably in a range of Expression (2).

$$0.0004 \ \mathrm{m}^2/\mathrm{liter} \ (\mathrm{L}) \leq (\mathrm{FA/MCC}) \leq 0.02 \ \mathrm{m}^2/\mathrm{L} \ ... \ (1)$$

$$0.001 \ \mathrm{m^2/L} \le (\mathrm{FA/MCC}) \le 0.01 \ \mathrm{m^2/L} \dots (2)$$

The maximum accommodation capacity MCC of the culture tank 14 is, for example, 50 L or more and 5,000 L or less. Therefore, for example, in a case where the maximum accommodation capacity MCC of the culture tank 14 is 50 L, the filtration area FA of the filter membranes 99 and 100 is 0.05 $m^2$ or more and 0.5 $m^2$ or less. In addition, for example, in a case where the maximum accommodation capacity MCC of the culture tank 14 is 5,000 L, the filtration area FA of the filter membranes 99 and 100 is 5 $m^2$ or more and 50 $m^2$ or less.

The intermediate container 60 is a bag formed by pouching entire peripheries of two resin-made sheets such as polyolefin and ethylene-vinyl acetate copolymer, and liquid-tightly bonding the two resin-made sheets. The intermediate container 60 is made of a single-use material which can be used only once, same as the tank 45 and the sampling container 64.

An inlet port 102 into which the culture supernatant liquid 29 from the culture section 10 flows is provided in a bottom portion 101 of the intermediate container 60. A flow-in tube 103 is attached to the inlet port 102, and a sterile connector 104 is provided at a distal end of the flow-in tube 103. A sterile connector 105 is also provided at a distal end of the coupling path 58. Through the sterile connectors 104 and 105, the coupling path 58 is sterilely connected to the flow-in tube 103, and thus sterilely connected to the intermediate container 60.

In addition, an outlet port 106 through which the culture supernatant liquid 29 flows out toward the purification section 12 is provided on the bottom portion 101 of the intermediate container 60. A flow-out tube 107 is attached to the outlet port 106, and a sterile connector 108 is provided at a distal end of the flow-out tube 107. A sterile connector 109 is also provided at a distal end of the coupling path 61. Through the sterile connectors 108 and 109, the coupling path 61 is sterilely connected to the flow-out tube 107, and thus sterilely connected to the intermediate container 60.

The intermediate container 60 is separated from the coupling paths 58 and 61 by removing the sterile connectors 104 and 108 from the sterile connectors 105 and 109. Sterile connectors 104 and 108 of a new intermediate container 60 are attached to the sterile connectors 105 and 109. As a result, the used intermediate container 60 is exchanged for the new intermediate container 60.

A flow rate of the culture supernatant liquid 29 flowing into the inlet port 102 by the delivery pump 35 is constant. In addition, a flow rate of the culture supernatant liquid 29 flowing out from the outlet port 106 by the first supply pump 62 is also constant. Furthermore, an outflow rate of the culture supernatant liquid 29 at the outlet port 106 is higher than an inflow rate of the culture supernatant liquid 29 at the inlet port 102. It is sufficient that the outflow rate of the culture supernatant liquid 29 at the outlet port 106 is 1% or more and 30% or less, preferably 2% or more and 20% or less, higher than the inflow rate of the culture supernatant liquid 29 at the inlet port 102.

A hanging tool 111 is attached to an upper portion 110 of the intermediate container 60. The intermediate container 60 can be used by hanging the intermediate container 60 on a hook 112 using the hanging tool 111. An electronic spring scale 113 is attached to the hook 112. The electronic spring scale 113 measures a weight of the intermediate container 60 in a state of being suspended from the hook 112 to measure a liquid amount of the culture supernatant liquid 29 in the intermediate container 60. A weight of the empty intermediate container 60 and a weight of the hook 112 are stored in advance in the electronic spring scale 113.

The electronic spring scale 113 calculates the liquid amount of the culture supernatant liquid 29 in the intermediate container 60 from the measured weight of the intermediate container 60 by subtracting the weight of the empty intermediate container 60 and the weight of the hook 112, and outputs the calculated weight as the measurement result. The electronic spring scale 113 transmits the measurement result of the liquid amount to the control device 13. The electronic spring scale 113 is further attached to a hook of a hanging stand (not shown). The electronic spring scale 113 may output the weight of the intermediate container 60 as the measurement result to the control device 13, and the control device 13 may perform conversion of the weight into the liquid amount. Alternatively, the weight of the intermediate container 60 itself may be treated as the liquid amount of the culture supernatant liquid 29.

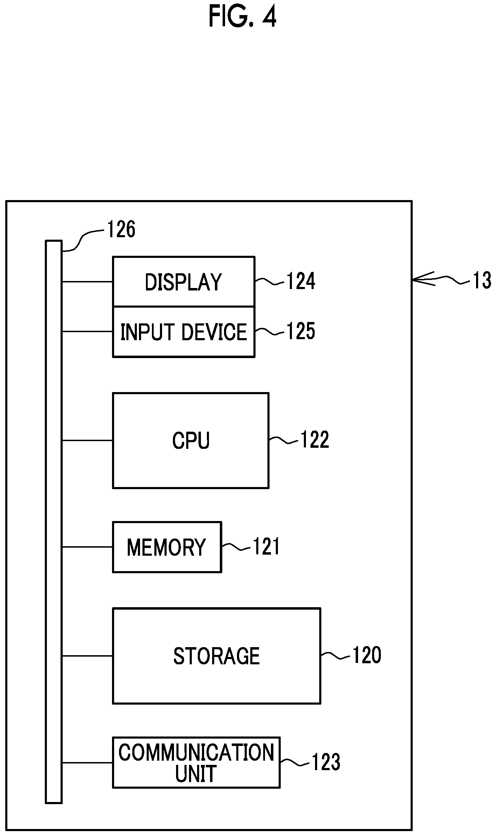

As an example shown in Fig. 4, a computer constituting the control device 13 includes a storage 120, a memory 121, a central processing unit (CPU) 122, a communication unit 123, a display 124, and an input device 125. These units are connected to one another through a bus line 126.

The storage 120 is a hard disk drive that is provided in the computer constituting the control device 13 or is connected to the computer through a cable or a network. Alternatively, the storage 120 is a disk array in which a plurality of hard disk drives are connected. The storage 120 stores, for example, a control program, such as an operating system, various application programs, and various types of data associated with these programs. In addition, a solid state drive may be used instead of the hard disk drive.

The memory 121 is a work memory for the CPU 122 to perform processes. The CPU 122 loads the program stored in the storage 120 to the memory 121 and performs a process corresponding to the program. As a result, the CPU 122 integrally controls each unit of the computer. The memory 121 may be incorporated in the CPU 122.

The communication unit 123 controls transmission of various information to an external apparatus. The display 124 displays various screens. The various screens have operation functions by a graphical user interface (GUI). The computer constituting the control device 13 receives operation instructions input from the input device 125 through various screens. The input device 125 is, for example, a keyboard, a mouse, a touch panel, and a microphone for voice input.

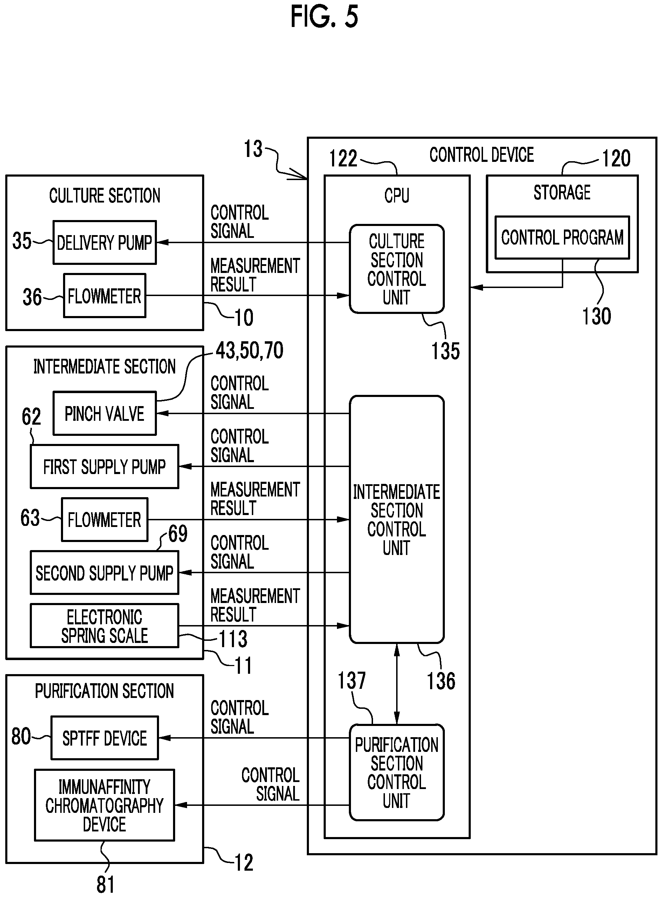

As an example shown in Fig. 5, a control program 130 is stored in the storage 120 of the control device 13. The control program 130 is an application program for causing the computer to function as the control device 13. In addition, the storage 120 stores data of various types of screens to be displayed on the display 124.

In a case where the control program 130 is activated, the CPU 122 of the computer constituting the control device 13 functions as a culture section control unit 135, an intermediate section control unit 136, and a purification section control unit 137, in cooperation with the memory 121 and the like. The culture section control unit 135 controls the operation of the culture section 10, the intermediate section control unit 136 controls the operation of the intermediate section 11, and the purification section control unit 137 controls the operation of the purification section 12.

The culture section control unit 135 receives the measurement result of the flow rate of the culture supernatant liquid 29 from the flowmeter 36. The culture section control unit 135 controls the flow rate of the culture supernatant liquid 29 based on the measurement result. More specifically, the culture section control unit 135 transmits, to the delivery pump 35, a control signal for setting the flow rate of the culture supernatant liquid 29 to a preset value. By such control, the flow rate of the culture supernatant liquid 29 flowing into the sterile filter 44 and the flow rate of the culture supernatant liquid 29 at the inlet port 102 of the intermediate container 60 are kept constant. In addition, the culture section control unit 135 also controls the supply amount of the culture liquid 16 through the culture liquid supply path 19, the supply amount of the gas containing oxygen through the sparger 23 and the gas supply path 24, the rotation speed of the stirring blade 25, and the like.

The intermediate section control unit 136 transmits a control signal to the pinch valves 43, 50, and 70. The control signal is a signal for transmitting any one of an instruction not to flow the current to the coil in the pinch valve 43 or the like, or an instruction to flow the current to the coil. In a case where the control signal for transmitting the instruction not to flow the current to the coil is received, the pinch valve 43 or the like opens the flow passage. On the other hand, in a case where the control signal for transmitting the instruction to flow the current to the coil is received, the pinch valve 43 or the like closes the flow passage. Hereinafter, the control signal for transmitting the instruction not to flow the current to the coil is denoted by a control signal (open). In addition, the control signal for transmitting the instruction to flow the current to the coil is denoted by a control signal (close).

The intermediate section control unit 136 receives the measurement result of the flow rate of the culture supernatant liquid 29 from the flowmeter 63. The intermediate section control unit 136 controls the flow rate of the culture supernatant liquid 29 based on the measurement result. More specifically, the intermediate section control unit 136 transmits, to the first supply pump 62, a control signal for setting the flow rate of the culture supernatant liquid 29 to a preset value. By such control, the flow rate of the culture supernatant liquid 29 at the outlet port 106 is kept constant.

In a case where the sampling of the culture supernatant liquid 29 for sampling is instructed through the input device 125, the intermediate section control unit 136 transmits a control signal to the second supply pump 69 to operate the second supply pump 69. In addition, the intermediate section control unit 136 transmits the control signal (open) to the pinch valve 70. As a result, the culture supernatant liquid 29 flows into the sampling container 64.

The intermediate section control unit 136 receives, from the electronic spring scale 113, the measurement result of the liquid amount of the culture supernatant liquid 29 in the intermediate container 60. The intermediate section control unit 136 operates or stops the first supply pump 62 based on the measurement result of the liquid amount. The intermediate section control unit 136 transmits a signal indicating that the first supply pump 62 is operated to the purification section control unit 137. In addition, the intermediate section control unit 136 transmits a signal indicating that the operation of the first supply pump 62 is stopped to the purification section control unit 137.

The purification section control unit 137 transmits the control signal to the SPTFF device 80 and the immunaffinity chromatography device 81. The built-in pump, valve, and the like of the SPTFF device 80 and the immunaffinity chromatography device 81 are operated in response to the control signal. In addition, although not shown, the purification section control unit 137 also transmits a control signal to the ion affinity chromatography device 86.

In addition, although not shown, the purification section control unit 137 receives an error signal from the SPTFF device 80 or the immunaffinity chromatography device 81. The error signal is issued from the SPTFF device 80 or the immunaffinity chromatography device 81 in a case where a malfunction occurs in the SPTFF device 80 or the immunaffinity chromatography device 81. The purification section control unit 137 also receives an error signal from the ion affinity chromatography device 86. The malfunction is, for example, a failure of a pump in the SPTFF device 80 or the immunaffinity chromatography device 81, or clogging of the column in the immunaffinity chromatography device 81. The purification section control unit 137 transmits the error signal to the intermediate section control unit 136. Furthermore, although not shown, in a case where the malfunction is resolved, a notification signal indicating the fact is issued from the SPTFF device 80, the immunaffinity chromatography device 81, or the ion affinity chromatography device 86 toward the purification section control unit 137.

In a case where the error signal is received from the purification section control unit 137, the intermediate section control unit 136 transmits the control signal (close) to the pinch valve 43 and then transmits the control signal (open) to the pinch valve 50. As a result, the flow passage of the culture supernatant liquid 29 is switched from the coupling path 41 to the branch path 42. That is, the intermediate section control unit 136 performs control of switching the flow passage of the culture supernatant liquid 29 from the coupling path 41 to the branch path 42 in a case where the error signal indicating that the malfunction has occurred in the purification section 12 is output. The culture supernatant liquid 29 flows into the tank 45 through the branch path 42, the flow-in tube 48, and the inlet port 47. In a case where the malfunction in the purification section 12 is resolved and the notification signal indicating the fact is received from the SPTFF device 80 and the like, the intermediate section control unit 136 transmits the control signal (close) to the pinch valve 50 and then transmits the control signal (open) to the pinch valve 43, and performs control of returning the flow passage of the culture supernatant liquid 29 from the branch path 42 to the coupling path 41.

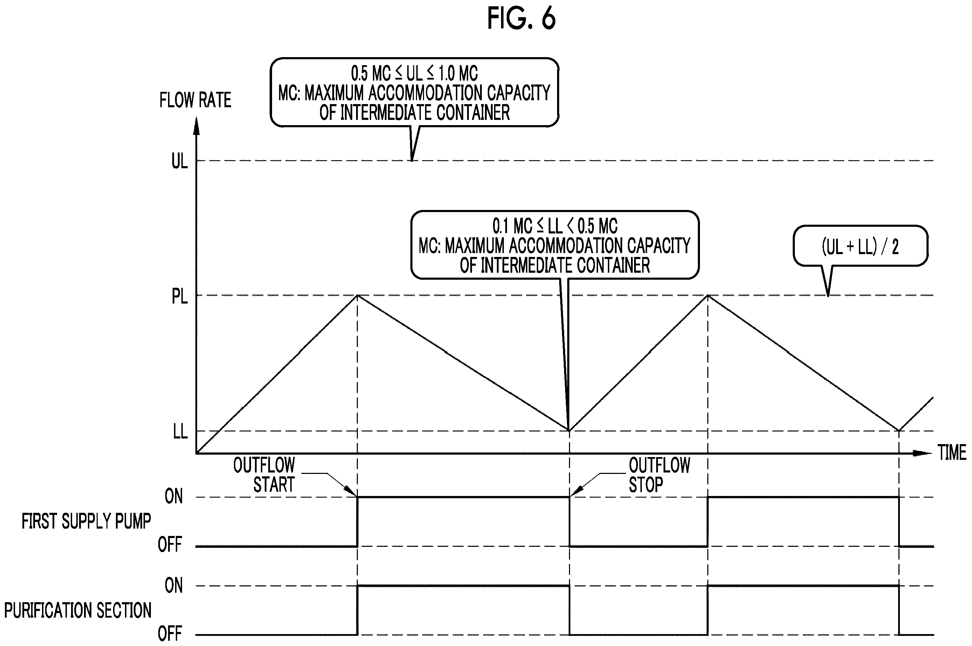

As an example shown in Fig. 6, in a case where it is detected that the liquid amount has reached a preset amount PL between an upper limit storage amount UL and a lower limit storage amount LL of the intermediate container 60 based on the measurement result of the liquid amount of the culture supernatant liquid 29 in the intermediate container 60, the intermediate section control unit 136 operates (turns on) the first supply pump 62 to start the outflow of the culture supernatant liquid 29 through the outlet port 106. In a case where a signal indicating the start of the operation of the first supply pump 62 is received from the intermediate section control unit 136, the purification section control unit 137 operates (turns on) the purification section 12.

On the other hand, in a case where it is detected that the liquid amount has reached the lower limit storage amount LL based on the measurement result of the liquid amount of the culture supernatant liquid 29 in the intermediate container 60, the intermediate section control unit 136 stops (turns off) the operation of the first supply pump 62 to stop the outflow of the culture supernatant liquid 29 through the outlet port 106. In a case where a signal indicating the stop of the operation of the first supply pump 62 is received from the intermediate section control unit 136, the purification section control unit 137 stops (turns off) the operation of the purification section 12. That is, the purification section 12 performs intermittent operation of operating while the culture supernatant liquid 29 is flowing out from the outlet port 106 and stopping the operation while the culture supernatant liquid 29 is not flowing out from the outlet port 106.

In a case where the weight of the intermediate container 60 itself is treated as the liquid amount of the culture supernatant liquid 29, the lower limit storage amount LL, the upper limit storage amount UL, and the set amount PL are also obtained from the weight of the intermediate container 60. For example, 3 kg is set as the lower limit storage amount LL, 7 kg is set as the upper limit storage amount UL, and 5 kg is set as the set amount PL. In a case where it is detected that the weight of the intermediate container 60 has reached 5 kg, the intermediate section control unit 136 starts the outflow of the culture supernatant liquid 29; and in a case where it is detected that the weight of the intermediate container 60 has reached 3 kg, the intermediate section control unit 136 stops the outflow of the culture supernatant liquid 29.

As described above, the flow rate of the culture supernatant liquid 29 from the culture section 10 to the inlet port 102 is constant. Therefore, the liquid amount of the culture supernatant liquid 29 increases linearly. In addition, as described above, the flow rate of the culture supernatant liquid 29 from the outlet port 106 to the purification section 12 is also constant. Therefore, the liquid amount of the culture supernatant liquid 29 decreases linearly. A slope of a straight line indicating the increase in liquid amount of the culture supernatant liquid 29 represents the increase amount of the liquid amount of the culture supernatant liquid 29 per unit time, that is, the inflow rate of the culture supernatant liquid 29 at the inlet port 102. On the other hand, a slope of a straight line indicating the decrease in liquid amount of the culture supernatant liquid 29 represents the decrease amount of the liquid amount of the culture supernatant liquid 29 per unit time, but does not match the outflow rate of the culture supernatant liquid 29 at the outlet port 106. This is because the culture supernatant liquid 29 continues to flow at the inlet port 102 even during the outflow of the culture supernatant liquid 29 from the outlet port 106.

As described above, the outflow rate of the culture supernatant liquid 29 at the outlet port 106 is higher than the inflow rate of the culture supernatant liquid 29 at the inlet port 102. Therefore, in a case where the amount of the culture supernatant liquid 29 flowing at the inlet port 102 is subtracted during the outflow of the culture supernatant liquid 29 from the outlet port 106, the slope of the straight line indicating the decrease in liquid amount of the culture supernatant liquid 29 is steeper than the slope of the straight line indicating the increase in liquid amount of the culture supernatant liquid 29. However, in reality, the amount of the culture supernatant liquid 29 flowing at the inlet port 102 during the outflow of the culture supernatant liquid 29 from the outlet port 106 is also included. In addition, a difference between the inflow rate of the culture supernatant liquid 29 at the inlet port 102 and the outflow rate of the culture supernatant liquid 29 at the outlet port 106 is slight. Therefore, the slope of the straight line indicating the decrease in liquid amount of the culture supernatant liquid 29 is more gentle than the slope of the straight line indicating the increase in liquid amount of the culture supernatant liquid 29 as shown in the drawing.

Here, the "flow rate of the culture supernatant liquid 29 from the outlet port 106 to the purification section 12 is constant" means that the flow rate is constant during the time from the start of the outflow of the culture supernatant liquid 29 through the outlet port 106 by operating the first supply pump 62 by the intermediate section control unit 136 to the stop of the outflow of the culture supernatant liquid 29 through the outlet port 106 by stopping the operation of the first supply pump 62.

In a case where the maximum accommodation capacity of the intermediate container 60 is denoted by MC, the upper limit storage amount UL of the intermediate container 60 is in a range of Expression (3).

$$0.5 \, \text{MC} \leq \text{UL} \leq 1.0 \, \text{MC} \, ... \, (3)$$

In this way, the upper limit storage amount UL does not necessarily match the maximum accommodation capacity MC.

In addition, the lower limit storage amount LL of the intermediate container 60 is in a range of Expression (4).

$$0.1 \text{MC} \leq \text{LL} < 0.5 \text{MC} \, ... \, (4)$$

In this way, the lower limit storage amount LL is a value larger than 0.

The set amount PL is, for example, represented by Expression (5).

$$\text{PL} = (\text{UL} + \text{LL})/2 \, ... \, (5)$$

Therefore, for example, in a case in which the upper limit storage amount UL is 1.0 MC and the lower limit storage amount LL is 0.2 MC, PL = (1.0 MC + 0.2 MC)/2 = 0.6 MC. The set amount PL is not limited to Expression (5). PL = 0.5 MC or PL = 0.9 MC may be used. The set amount PL may be set to be substantially the same as the maximum accommodation capacity MC, such as PL = 0.9999 MC.

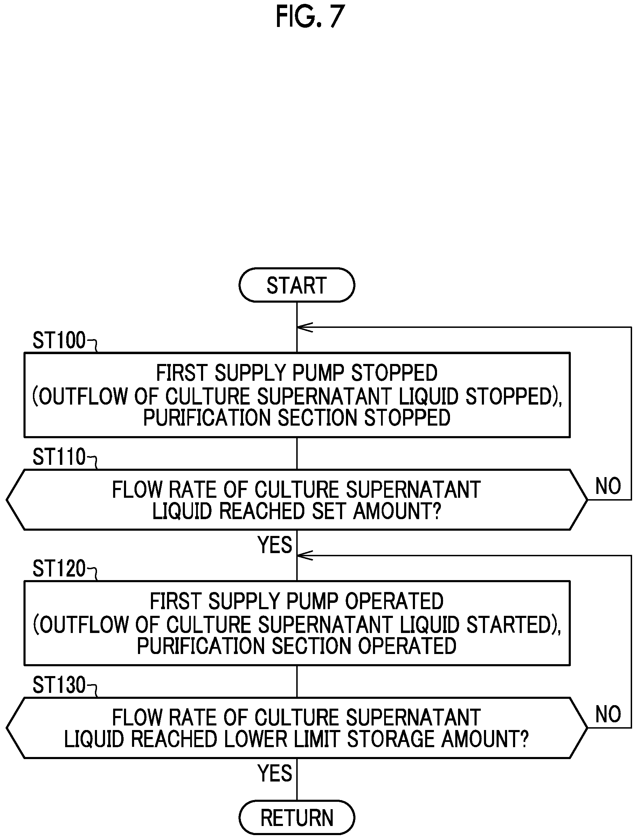

Next, an operation according to the above-described configuration will be described with reference to a flowchart illustrated in Fig. 7 as an example. First, the antibody-producing cells 17 are seeded in the culture tank 14 of the culture section 10, and are cultured in the culture liquid 16. As a result, the antibody 18 is produced from the antibody-producing cells 17, and is dispersed in the culture liquid 16.

The culture liquid 16 is sent to the cell separation filter 15 through the culture liquid delivery and recovery path 22 and the inlet and outlet 27. The antibody-producing cells 17 are removed from the culture liquid 16 by the filter membrane 28 of the cell separation filter 15, and the culture supernatant liquid 29 is obtained. The culture supernatant liquid 29 flows out from the outlet 33 to the coupling path 34, and is sent downstream by the delivery pump 35. In this case, the control signal according to the measurement result of the flowmeter 36 is transmitted from the culture section control unit 135 to the delivery pump 35, whereby the flow rate of the culture supernatant liquid 29 is controlled to be constant based on the measurement result of the flowmeter 36.

The culture supernatant liquid 29 flows from the coupling path 34 to the coupling path 41 and passes through the pinch valve 43 and the sterile filter 44. By passing through the sterile filter 44, impurities other than the antibody 18 in the culture supernatant liquid 29 are removed.

The culture supernatant liquid 29 which has passed through the sterile filter 44 flows into the inlet port 102 of the intermediate container 60 through the coupling path 58 and the flow-in tube 103, and flows into the intermediate container 60 through the inlet port 102. As a result, the culture supernatant liquid 29 is stored in the intermediate container 60.

As shown in Fig. 3, the liquid amount of the culture supernatant liquid 29 in the intermediate container 60 is measured by the electronic spring scale 113. The measurement result of the liquid amount of the culture supernatant liquid 29 is transmitted from the electronic spring scale 113 to the intermediate section control unit 136.

As shown in Fig. 6, until the liquid amount of the culture supernatant liquid 29 reaches the set amount PL (NO in the step ST110 of Fig. 7), the operation of the first supply pump 62 is stopped by the intermediate section control unit 136, and the operation of the purification section 12 is stopped by the purification section control unit 137 (step ST100). Here, in a case where it is detected that the liquid amount of the culture supernatant liquid 29 has reached the set amount PL (YES in the step ST110), the first supply pump 62 is operated by the intermediate section control unit 136, and the outflow of the culture supernatant liquid 29 through the outlet port 106 is started. In addition, the purification section 12 is operated by the purification section control unit 137 (step ST120). In the purification section 12, the antibody 18 in the culture supernatant liquid 29 is continuously purified by the SPTFF device 80 and the immunaffinity chromatography device 81, and the active pharmaceutical ingredient 89 of a biopharmaceutical is finally obtained.

As long as the liquid amount of the culture supernatant liquid 29 does not reach the lower limit storage amount LL (NO in the step ST130), the operation of the first supply pump 62 is continued by the intermediate section control unit 136, and the operation of the purification section 12 is continued by the purification section control unit 137 (step ST120). Here, in a case where it is detected that the liquid amount of the culture supernatant liquid 29 has reached the lower limit storage amount LL (YES in the step ST130), the operation of the first supply pump 62 is stopped by the intermediate section control unit 136, and the outflow of the culture supernatant liquid 29 through the outlet port 106 is stopped. In addition, the operation of the purification section 12 is stopped by the purification section control unit 137 (step ST100).

In a case where no malfunction occurs in the purification section 12, the control signal (open) is transmitted from the intermediate section control unit 136 to the pinch valve 43, and the control signal (close) is transmitted from the intermediate section control unit 136 to the pinch valve 50. As a result, the coupling path 41 is opened, and the branch path 42 is closed. In this way, the culture supernatant liquid 29 flows from the coupling path 34 to the coupling path 41.

On the other hand, in a case where a malfunction occurs in the purification section 12, an error signal is issued from the SPTFF device 80 or the like to the purification section control unit 137. The error signal is transmitted from the purification section control unit 137 to the intermediate section control unit 136. In this case, from the intermediate section control unit 136, the control signal (close) is transmitted to the pinch valve 43, and then the control signal (open) is transmitted to the pinch valve 50. As a result, the coupling path 41 is closed, and the branch path 42 is opened. In this way, the culture supernatant liquid 29 flows into the tank 45. The state in which the culture supernatant liquid 29 flows into the tank 45 and then temporarily stored is continued until the malfunction in the purification section 12 is resolved.

In a case where the malfunction in the purification section 12 is resolved, the control signal (close) is transmitted from the intermediate section control unit 136 to the pinch valve 50, and then the control signal (open) is transmitted to the pinch valve 43. As a result, the coupling path 41 is opened again, the branch path 42 is closed, and the culture supernatant liquid 29 flows from the coupling path 34 to the coupling path 41.

In a case where the sampling of the culture supernatant liquid 29 for sampling is instructed through the input device 125, a control signal is transmitted from the intermediate section control unit 136 to the second supply pump 69 to operate the second supply pump 69. In addition, the control signal (open) is transmitted from the intermediate section control unit 136 to the pinch valve 70. As a result, the culture supernatant liquid 29 flows into the sampling container 64.

As described above, in the method for producing of an active pharmaceutical ingredient of a biopharmaceutical according to the technology of the present disclosure, the culture section 10 and the purification section 12 are connected to each other by the coupling paths 34, 41, 58, and 61. In the culture section 10, a culture step of culturing the antibody-producing cells 17 in the culture liquid 16 stored in the culture tank 14 and obtaining the culture supernatant liquid 29 containing the antibody 18 by removing the antibody-producing cells 17 from the culture liquid 16 using the cell separation filter 15 is performed. In the purification section 12, a purification step of purifying the antibody 18 from the culture supernatant liquid 29 to obtain the active pharmaceutical ingredient 89 of a biopharmaceutical is performed. The purification section 12 includes the purification step using the purification filter 82 of the SPTFF method. The sterile filter 44 having a pore diameter larger than that of the cell separation filter 15 is provided in the coupling path 41, and the culture supernatant liquid 29 flowing from the culture section 10 to the purification section 12 through the coupling path 41 is filtered with the sterile filter 44.

Since the purification section 12 includes the purification step using the purification filter 82 of the SPTFF method, the following effects are obtained. That is, according to the purification step using the purification filter 82 of the SPTFF method, the antibody 18 can be purified by a single circulation of the culture supernatant liquid 29. In the circulating purification step in the related art, an additional pump and tank are required, and the work is also complicated. In addition, since the configuration is complicated, the risk of contamination is also high. On the other hand, in the purification step using the purification filter 82 of the SPTFF method, an additional pump and tank are not required, and the workability is also favorable. In addition, since the configuration is simple, the risk of contamination is also low. In addition, with the purification filter 82 of the SPTFF method, the impurities in the culture supernatant liquid 29 can be effectively removed. Therefore, the load on the subsequent immunoaffinity chromatography device 81 is reduced, and the risk of malfunction such as clogging of the column of the immunoaffinity chromatography device 81 can be reduced. Accordingly, it is possible to suppress a decrease in maintainability.

In addition, the sterile filter 44 has a larger pore diameter than the cell separation filter 15. In other words, the cell separation filter 15 has a smaller pore diameter than the sterile filter 44. Therefore, most of the impurities in the culture supernatant liquid 29 are removed by the cell separation filter 15. As a result, the load applied to the sterile filter 44 located downstream is reduced, and the malfunction such as clogging in the sterile filter 44 is less likely to occur. Therefore, it is possible to suppress the decrease in maintainability. In addition, the malfunction such as clogging in the sterile filter 44 frequently occurs, and continuous purification of the antibody 18 by the purification section 12 can be frequently stopped. Specifically, the continuous purification can be performed for several days to several tens of days without stopping even once due to the malfunction of the sterile filter 44.

The sterile filter 44 may have a two-layer structure filter membrane satisfying the following condition, in addition to the condition of having a pore diameter larger than that of the cell separation filter 15.

Condition: even in a case where the indicator bacteria are loaded at $10^7$ /cm$^2$ on the upstream side, it is possible to obtain a sterile liquid on the downstream side.

As shown in Fig. 1, the subsequent step of the purification step using the purification filter 82 of the SPTFF method is a single-column purification step using the immunoaffinity chromatography device 81. Therefore, it is possible to reduce the risk of occurrence of the malfunction as compared with a case in which the purification step is performed using a plurality of chromatography devices each having a different column.

With the purification filter 82 of the SPTFF method, the antibody 18 can be sufficiently concentrated in a short time as compared with a case of using a chromatography device. Therefore, even in a case where the subsequent of the purification step using the purification filter 82 of the SPTFF method is the purification step using the single-column immunaffinity chromatography device 81, that is, even in a case where the subsequent step of the purification step using the purification filter 82 of the SPTFF method is not a purification step using a plurality of chromatography devices each having a different column, it is possible to obtain the antibody 18 with a high purity.

In addition, the purification filter 82 of the SPTFF method does not require maintenance such as stopping the inflow of the culture supernatant liquid 29 and washing, as in the column of the chromatography device, and the culture supernatant liquid 29 can be continuously flowed. Therefore, the aspect of operating the purification section 12 while the culture supernatant liquid 29 is flowing out from the outlet port 106 is compatible.

As shown in Fig. 3, in a case where the maximum accommodation capacity of the culture tank 14 is denoted by MCC and a filtration area of the sterile filter 44 is denoted by FA, $0.001 \text{ m}^2/\text{L} \leq (\text{FA/MCC}) \leq 0.01 \text{m}^2/\text{L}$. Therefore, the culture supernatant liquid 29 can be filtered using the sterile filter 44 having a relatively small filtration area FA with respect to the maximum accommodation capacity MCC of the culture tank 14.

As shown in Fig. 1, the pore diameter of the sterile filter 44 is larger than the pore diameter of the purification filter 82 of the SPTFF device 80. Therefore, the load applied to the sterile filter 44 is further reduced, and the malfunction such as clogging in the sterile filter 44 is less likely to occur. Therefore, it is possible to further suppress the decrease in maintainability. In addition, as shown in Fig. 1, the pore diameter of the sterile filter 44 is 0.1 $\mu$m or more and 1.0 $\mu$m or less. Therefore, the impurities in the culture supernatant liquid 29 can be effectively captured.

As shown in Fig. 3, the sterile filter 44 has a structure in which a flat filter paper is folded. The sterile filter 44 having a structure in which a flat filter paper is folded is relatively inexpensive. Therefore, the cost of the sterile filter 44 can be reduced. In addition, a material of the sterile filter 44 is polyethersulfone. The polyethersulfone has high filterability and is less likely to be clogged. Therefore, it is possible to further suppress the decrease in maintainability.

As shown in Fig. 1, the culture supernatant liquid 29 is sent from the culture section 10 toward the purification section 12 by the delivery pump 35 provided upstream of the sterile filter 44 in the coupling line such as the coupling path 34. As compared with a case in which the delivery pump 35 is provided downstream of the sterile filter 44, it is possible to reduce the risk of malfunction that the sterile filter 44 is clogged due to air bubbles from the delivery pump 35.

As shown in Fig. 1, the culture supernatant liquid 29 is temporarily stored in the intermediate container 60 provided downstream of the sterile filter 44 in the coupling line such as the coupling path 34. Therefore, the culture supernatant liquid 29 can be continuously purified by the purification section 12 without interruption.

As shown in Fig. 3, the flow rate of the culture supernatant liquid 29 to the sterile filter 44 is constant. Therefore, the state of the culture supernatant liquid 29 flowing into the sterile filter 44 can be kept constant.

As shown in Fig. 2, in a case where the integrated flow rate of the culture supernatant liquid 29 to the sterile filter 44 has reached a set amount set to be 1,000 $\text{L/m}^2$ to 10,000 $\text{L/m}^2$, the sterile filter 44 is exchanged. Therefore, the sterile filter 44 in which the integrated flow rate has reached the set amount and the filtration performance has deteriorated is not used continuously, and the sterile filter 44 having always good filtration performance can be used.

As shown in Fig. 1, the culture supernatant liquid 29 is allowed to flow into the sampling container 64 connected downstream of the sterile filter 44 in the coupling path 41. In a case where the sampling container 64 is connected upstream of the sterile filter 44, the bacteria or the like may enter the culture section 10 from the branch path 59 including the sampling container 64 and the like, and the culture section 10 may be contaminated. However, in the present example, since the sterile filter 44 is provided to prevent the entry of bacteria or the like from the branch path 59, a sterile state on the upstream side of the sterile filter 44 can be maintained.

In addition, as shown in Fig. 1, the culture supernatant liquid 29 is allowed to flow into the sampling container 64 using the second supply pump 69 provided in the branch path 59 connecting the coupling paths 41 and 58 and the sampling container 64. Therefore, the culture supernatant liquid 29 can be reliably allowed to flow into the sampling container 64. In addition, since the second supply pump 69 plays a role of a valve, it is possible to suppress the entry of bacteria or the like from the downstream side of the second supply pump 69.

The biopharmaceutical containing the antibody 18 as an active ingredient is called an antibody pharmaceutical, and is widely used for the treatment of rare diseases such as hemophilia and Crohn's disease, as well as the treatment of chronic diseases such as cancer, diabetes, and rheumatoid arthritis. Therefore, according to the present example in which the cell is the antibody-producing cells 17 derived from CHO cells and the product is the protein, particularly the antibody 18, it is possible to contribute to the development of antibody pharmaceuticals which are widely used for the treatment of various diseases.

[Second embodiment]

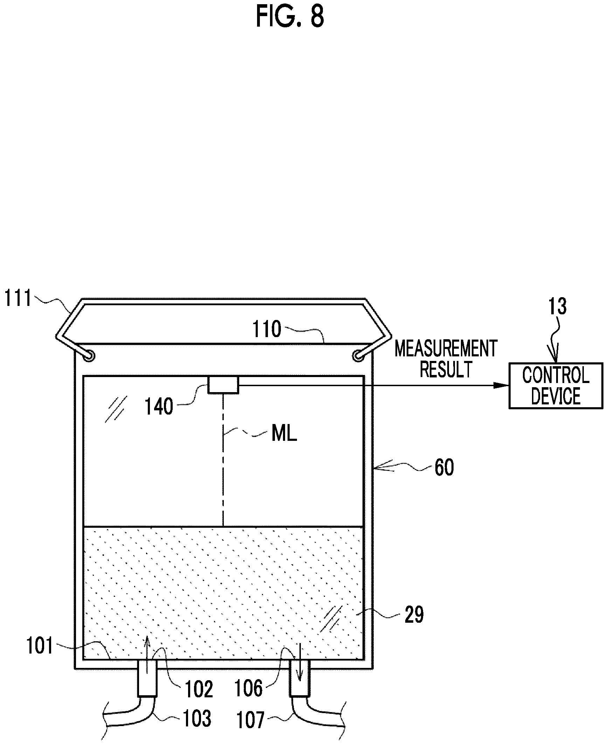

As an example shown in Fig. 8, in the second embodiment, a liquid level sensor 140 is provided on the upper portion 110 of the intermediate container 60. The liquid level sensor 140 detects a position of a liquid surface of the culture supernatant liquid 29 in the intermediate container 60, whereby the liquid level sensor 140 measures the liquid amount of the culture supernatant liquid 29 in the intermediate container 60.

The liquid level sensor 140 is, for example, a reflective type photo sensor (also referred to as a photoreflector) which radiates a measurement light ML toward the liquid surface of the culture supernatant liquid 29 and receives reflected light of the measurement light ML from the liquid surface of the culture supernatant liquid 29. The liquid level sensor 140 detects a position of the liquid surface of the culture supernatant liquid 29 by a difference in intensity of the reflected light received. The liquid level sensor 140 refers to a data table or the like, showing a relationship between the position of the liquid surface of the culture supernatant liquid 29 and the liquid amount of the culture supernatant liquid 29 in the intermediate container 60; converts the detected position of the liquid surface of the culture supernatant liquid 29 into the liquid amount of the culture supernatant liquid 29 in the intermediate container 60; and outputs the converted liquid amount as a measurement result. The liquid level sensor 140 transmits the measurement result of the liquid amount to the control device 13. Since the subsequent processing is the same as the processing of the first embodiment described above, the description thereof will be omitted. The liquid level sensor 140 may output the position of the liquid surface of the culture supernatant liquid 29 as the measurement result to the control device 13, and the control device 13 may perform the conversion of the position of the liquid surface of the culture supernatant liquid 29 into the liquid amount. Alternatively, as in the case of the weight of the intermediate container 60, the position of the liquid surface itself may be treated as the liquid amount of the culture supernatant liquid 29.

As described above, in the second embodiment, the liquid amount of the culture supernatant liquid 29 in the intermediate container 60 is measured by measuring the position of the liquid surface of the culture supernatant liquid 29. Therefore, even in a situation in which the measurement of the weight of the intermediate container 60 is difficult, the liquid amount of the culture supernatant liquid 29 in the intermediate container 60 can be measured. The liquid level sensor may be a liquid level sensor using ultrasound.

[Third embodiment]

In the first embodiment described above, the flow rate of the culture supernatant liquid 29 from the outlet port 106 to the purification section 12 is kept constant, but the present invention is not limited thereto.

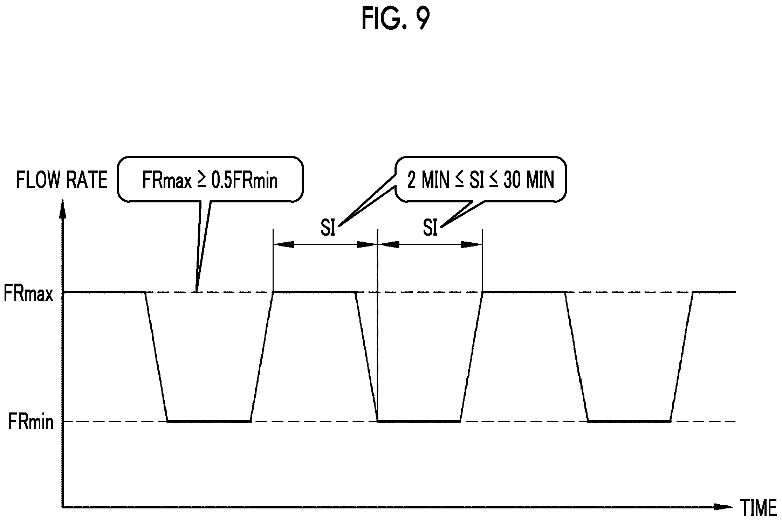

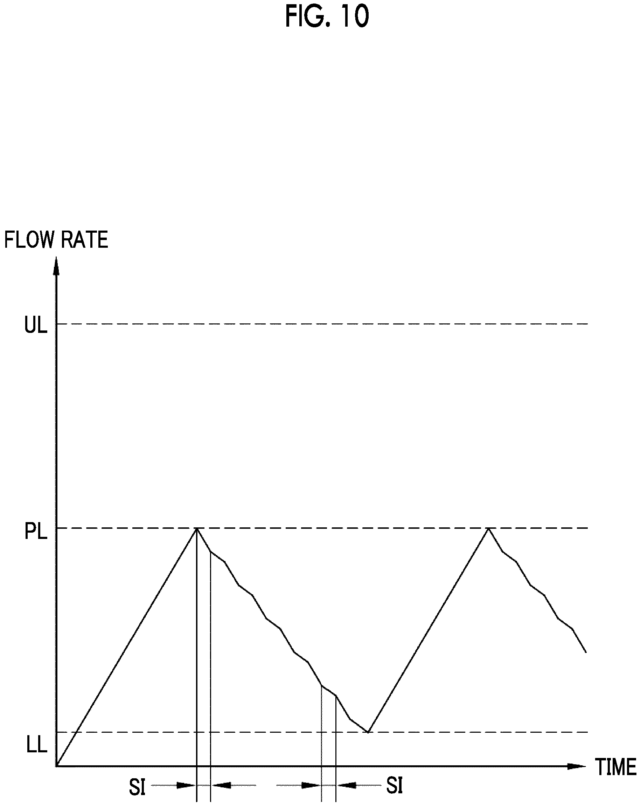

As an example shown in Fig. 9, in the third embodiment, the intermediate section control unit 136 controls the operation of the first supply pump 62 to vary the flow rate of the culture supernatant liquid 29 flowing into the purification filter 82 of the SPTFF device 80 at a set interval SI. The set interval SI is set to be 2 minutes to 30 minutes ($2 \text{ min} \leq SI \leq 30$ min). In addition, the maximum value FRmax of the flow rate of the culture supernatant liquid 29 is half or more of the minimum value FRmin ($FRmax \geq 0.5$ FRmin). That is, the flow rate of the culture supernatant liquid 29 flowing into the purification filter 82 is changed by 50% or more. In this case, the liquid amount of the culture supernatant liquid 29 in the intermediate container 60 decreases in a broken line shape, not in a straight line shape, as an example shown in Fig. 10.

As described above, in the third embodiment, the flow rate of the culture supernatant liquid 29 flowing into the purification filter 82 is varied by 50% or more at the set interval SI set to be 2 minutes to 30 minutes. By changing the flow rate of the culture supernatant liquid 29 flowing into the purification filter 82 in this manner, it is possible to expect a washing effect of washing away the impurities captured in the purification filter 82 by the inflow culture supernatant liquid 29. The purification filter 82 can be efficiently washed while purifying the antibody 18.

In a case where the set interval SI is less than 2 minutes, the washing effect is increased, but the variation of the flow rate of the culture supernatant liquid 29 is too frequent to sufficiently concentrate the antibody 18. On the other hand, in a case where the set interval SI is more than 30 minutes, the washing effect is reduced, and clogging in the purification filter 82 is likely to occur. In addition, even in a case where the width of the variation is less than 50%, the washing effect is still reduced, and the clogging in the purification filter 82 is likely to occur. Therefore, the set interval SI is set to be 2 minutes to 30 minutes, and the width of the variation of the flow rate is set to be 50% or more.

[Fourth embodiment]

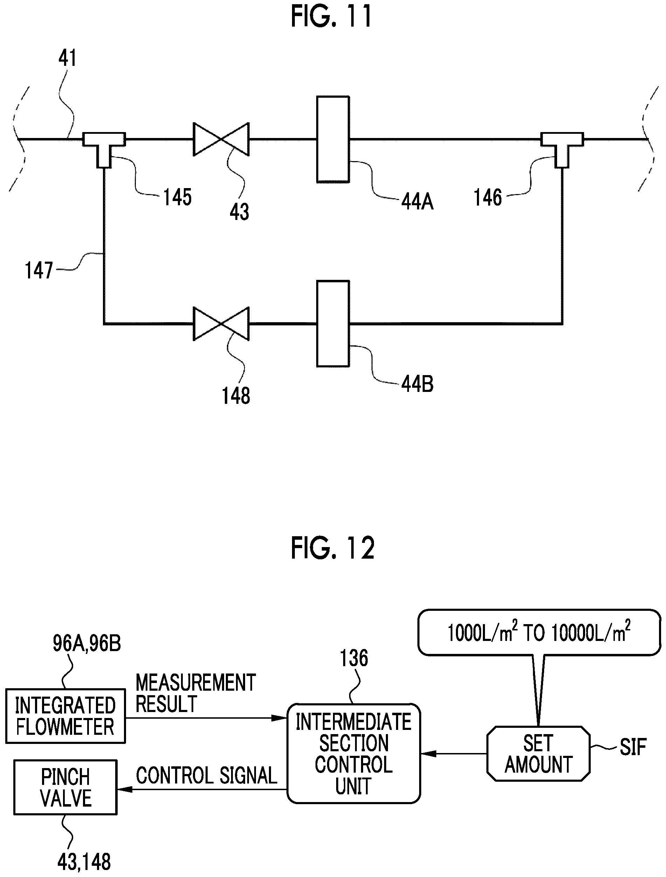

In each of the above-described embodiments, it is described that one sterile filter 44 is used, but the present disclosure is not limited thereto. As an example shown in Fig. 11, a plurality of sterile filters 44 may be used.

Fig. 11 shows an example in which two sterile filters 44A and 44B are used. In this case, three-way joints 145 and 146 are provided on the upstream side of the pinch valve 43 in the coupling path 41 and on the downstream side of the sterile filter 44A, respectively. A branch path 147 is connected to the three-way joints 145 and 146, and a pinch valve 148 and the sterile filter 44B are provided in the branch path 147. Although not shown, the sterile filter 44B is also sterilely connected to the branch path 147 through the sterile connectors 90, the sterile connector 92, and the like.

As an example shown in Fig. 12, in the present embodiment, measurement results of integrated flow rates of the culture supernatant liquid 29 from an integrated flowmeter 96A incorporated in the sterile filter 44A and an integrated flowmeter 96B incorporated in the sterile filter 44B are input to the intermediate section control unit 136. The intermediate section control unit 136 compares the measurement results of the integrated flow rates of the culture supernatant liquid 29 with a set amount SIF. The set amount SIF is set to be 1,000 L/m$^2$ to 10,000 L/m$^2$, as described in Fig. 2 of the first embodiment above. In a case where the measurement results of the integrated flow rates of the culture supernatant liquid 29 reach the set amount SIF, the intermediate section control unit 136 transmits the control signal to the pinch valves 43 and 148.

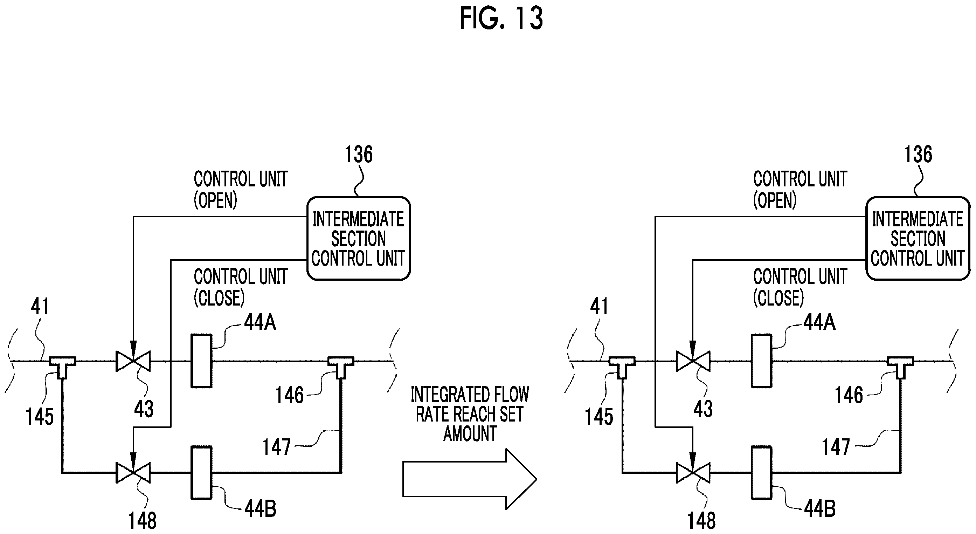

The left side of Fig. 13 shows a state in which the control signal (open) is transmitted from the intermediate section control unit 136 to the pinch valve 43 and the control signal (close) is transmitted to the pinch valve 148, and the culture supernatant liquid 29 is filtered by the sterile filter 44A of the coupling path 41. In the state shown in the left side of Fig. 13, in a case where the measurement result of the integrated flow rate of the culture supernatant liquid 29 from the integrated flowmeter 96A incorporated in the sterile filter 44A reaches the set amount SIF, the intermediate section control unit 136 transmits the control signal (close) to the pinch valve 43 and then transmits the control signal (open) to the pinch valve 148, as shown in the right side of Fig. 13. As a result, the flow passage of the culture supernatant liquid 29 is switched from the coupling path 41 to the branch path 147, and the culture supernatant liquid 29 flows into the sterile filter 44B of the branch path 147. That is, in a case where the integrated flow rate of the culture supernatant liquid 29 of one of the plurality of sterile filters 44 reaches the set amount SIF, the intermediate section control unit 136 performs switching for another sterile filter 44 to be used.

As described above, in the fourth embodiment, the sterile filter 44B connected in parallel to the coupling path 41 is used in addition to the sterile filter 44A provided in the coupling path 41. Therefore, the effort of exchanging the sterile filter 44 can be reduced. The number of the sterile filters 44 connected in parallel to the coupling path 41 is not limited to 1 as the example, and may be 2 or more.

[Fifth embodiment]

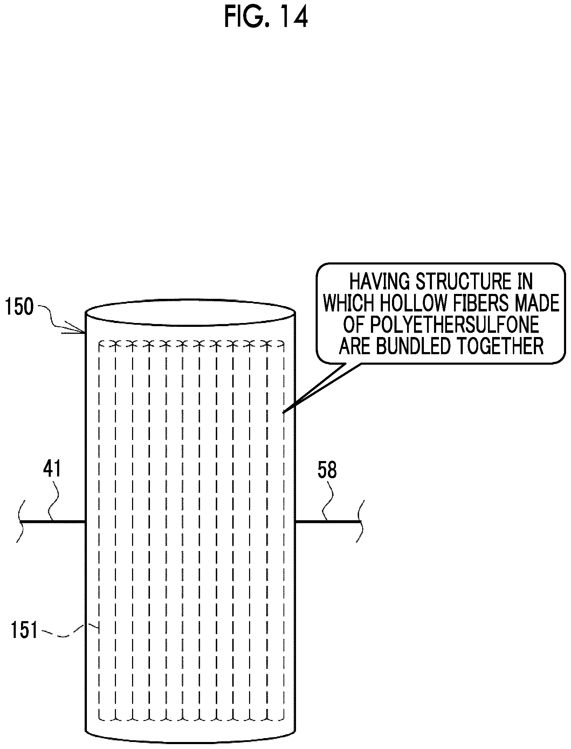

In the first embodiment described above, the sterile filter 44 including the filter membranes 99 and 100, having the structure in which a flat filter paper made of polyethersulfone is folded, has been described as an example, but the present invention is not limited thereto. As an example, a sterile filter 150 shown in Fig. 14 may be used. The sterile filter 150 has a filter membrane 151 having a structure in which hollow fibers made of polyethersulfone are bundled together. The sterile filter 150 having such a filter membrane 151 is called a hollow fiber filter.

The filter membrane 151 having the structure in which hollow fibers are bundled together has a washing effect. Therefore, with the sterile filter 150, the frequency of replacement can be reduced as compared with the sterile filter 44 having the filter membranes 99 and 100, in which a flat filter paper is folded. The filter membrane 151 may also have a two-layer structure as in the filter membranes 99 and 100 of the first embodiment described above.

The sterile connector 46 or the like has been mentioned as the sterile connection means, but the present invention is not limited thereto. The connection may be made in a sterile manner by thermal welding. As the thermal welding, for example, Biowelder (Internet Uniform Resource Locator (URL): https://premium.ipros.jp/sartorius-stedim/ product/detail/2000608451/?hub=163&categoryId= 52772) manufactured by Sartorius Stedim Japan Co., Ltd. is used.

Although the pinch valves 43 and the like are mentioned as the no liquid-contact type valve, the present invention is not limited thereto. A non-electric (manual) tube clamp may be used instead of the pinch valve 43 or the like. As the tube clamp, a screw type, a crocodile mouth type, a roller type, a ratchet type, or the like can be adopted.

In a case where a non-electrified no liquid-contact type valve such as the tube clamp is used, an indicator such as a warning light may be provided in the purification section 12, and the user may be notified of the occurrence of the malfunction in the purification section 12 by the indicator. In this case, the SPTFF device 80 or the immunaffinity chromatography device 81 may not issue the error signal to the purification section control unit 137.

The product is not limited to the antibody 18. The product is not particularly limited as long as it is the active pharmaceutical ingredient 89 of a biopharmaceutical, and may be a protein, a peptide, a nucleic acid (DNA or ribonucleic acid (RNA)), a lipid, a virus, a virus subunit, a virus-like particle, or the like, other than the antibody 18.

In each of the above-described embodiments, for example, as hardware structures of processing units that execute various kinds of processing, such as the culture section control unit 135, the intermediate section control unit 136, and the purification section control unit 137, various processors shown below can be used. The various processors include, for example, the CPU 122 which is a general-purpose processor executing software (control program 130) to function as various processors as described above, a programmable logic device (PLD), such as a field programmable gate array (FPGA), which is a processor whose circuit configuration can be changed after manufacture, and a dedicated electric circuit, such as an application specific integrated circuit (ASIC), which is a processor having a dedicated circuit configuration designed to perform a specific process.

One processing unit may be configured by one of the various types of processors or may be configured by a combination of two or more processors of the same type or different types (for example, a combination of a plurality of FPGAs and/or a combination of a CPU and an FPGA). In addition, a plurality of processing units may be configured by one processor.

As an example of configuring the plurality of processing units with one processor, first, there is a form in which one processor is configured by a combination of one or more CPUs and software and the processor functions as the plurality of processing units, as represented by computers such as a client and a server. A second example of the configuration is an aspect in which a processor that implements the functions of the entire system including a plurality of processing units using one integrated circuit (IC) chip is used. A representative example of the aspect is a system-on-chip (SoC). In this manner, various processing units are configured by using one or more of the above-described various processors as hardware structures.

In addition, more specifically, an electric circuit (circuitry) in which circuit elements, such as semiconductor elements, are combined can be used as the hardware structure of these various processors.

In the technology of the present disclosure, the above-described various embodiments and/or various modification examples can be appropriately combined. It is needless to say that the technique of the present disclosure is not limited to each of the embodiments described above and various configurations can be employed without departing from the gist. Furthermore, the technology of the present disclosure extends to a storage medium that non-transitorily stores a program in addition to the program.

The contents described and shown above are detailed descriptions of portions according to the technology of the present disclosure and are merely examples of the technology of the present disclosure. For example, the above description of the configurations, functions, operations, and effects is the description of examples of the configurations, functions, operations, and effects of portions related to the technology of the present disclosure. Therefore, unnecessary portions may be deleted or new elements may be added or replaced in the above descriptions and illustrations without departing from the gist of the technology of the present disclosure. In addition, in order to avoid complication and facilitate understanding of portions according to the technology of the present disclosure, description related to common technical knowledge or the like that does not need to be particularly described for enabling implementation of the technology of the present disclosure is omitted in the contents described and shown above.

In the present specification, "A and/or B" has the same meaning as "at least one of A or B". That is, the "A and/or B" means only A, only B, or a combination of A and B. In addition, in the present specification, in a case where three or more matters are expressed by being connected by "and/or", the same concept as "A and/or B" is applied.

All documents, patent applications, and technical standards described in the present specification are incorporated herein by reference to the same extent as in a case of being specifically and individually noted that individual documents, patent applications, and technical standards are incorporated by reference.

## Claims

1. A method for producing an active pharmaceutical ingredient of a biopharmaceutical, the method comprising:

   connecting, through a coupling line, a culture section where a culture step, in which cells are cultured in a culture liquid stored in a culture tank and the cells are removed from the culture liquid using a cell separation filter to obtain a culture supernatant liquid containing a product of the cells, is performed and a purification section where a purification step, in which the product is purified from the culture supernatant liquid to obtain an active pharmaceutical ingredient of a biopharmaceutical, is performed, the purification step including a purification step using a purification filter of a single pass tangential flow filtration method;
   providing, in the coupling line, a sterile filter having a pore diameter larger than a pore diameter of the cell separation filter; and
   filtering, with the sterile filter, the culture supernatant liquid flowing from the culture section to the purification section through the coupling line.

2. The method for producing an active pharmaceutical ingredient of a biopharmaceutical according to claim 1, wherein a subsequent step of the purification step using the purification filter of the single pass tangential flow filtration method is a purification step using a chromatography device.

3. The method for producing an active pharmaceutical ingredient of a biopharmaceutical according to claim 2, wherein the chromatography device is a single column.

4. The method for producing an active pharmaceutical ingredient of a biopharmaceutical according to any one of claims 1 to 3, wherein a sterile filter connected in parallel to the coupling line is used in addition to the sterile filter provided in the coupling line.

5. The method for producing an active pharmaceutical ingredient of a biopharmaceutical according to any one of claims 1 to 4,

wherein, in a case where a maximum accommodation capacity of the culture tank is denoted by MCC and a filtration area of the sterile filter is denoted by FA, $0.001 \text{ m}^2/\text{L} \leq (\text{FA/MCC}) \leq 0.01 \text{m}^2/\text{L}$.

6. The method for producing an active pharmaceutical ingredient of a biopharmaceutical according to any one of claims 1 to 5,
wherein the pore diameter of the sterile filter is larger than a pore diameter of the purification filter of the single pass tangential flow filtration method.

7. The method for producing an active pharmaceutical ingredient of a biopharmaceutical according to any one of claims 1 to 6,
wherein the pore diameter of the sterile filter is 0.1 $\mu$m or more and 1.0 $\mu$m or less.

8. The method for producing an active pharmaceutical ingredient of a biopharmaceutical according to any one of claims 1 to 7,
wherein the sterile filter has a structure in which a flat filter paper is folded.

9. The method for producing an active pharmaceutical ingredient of a biopharmaceutical according to any one of claims 1 to 7,
wherein the sterile filter has a structure in which hollow fibers are bundled together.

10. The method for producing an active pharmaceutical ingredient of a biopharmaceutical according to any one of claims 1 to 9,
wherein a material of the sterile filter is polyethersulfone.

11. The method for producing an active pharmaceutical ingredient of a biopharmaceutical according to any one of claims 1 to 10,
wherein the culture supernatant liquid is sent from the culture section toward the purification section by a delivery pump provided upstream of the sterile filter in the coupling line.

12. The method for producing an active pharmaceutical ingredient of a biopharmaceutical according to any one of claims 1 to 11,
wherein the culture supernatant liquid is temporarily stored in an intermediate container provided downstream of the sterile filter in the coupling line.

13. The method for producing an active pharmaceutical ingredient of a biopharmaceutical according to any one of claims 1 to 12,
wherein a flow rate of the culture supernatant liquid to the sterile filter is constant.

14. The method for producing an active pharmaceutical ingredient of a biopharmaceutical according to any one of claims 1 to 13,
wherein, in a case where an integrated flow rate of the culture supernatant liquid to the sterile filter has reached a set amount set to be 1,000 $\text{L/m}^2$ to 10,000 $\text{L/m}^2$, the sterile filter is exchanged.

15. The method for producing an active pharmaceutical ingredient of a biopharmaceutical according to any one of claims 1 to 14,
wherein a flow rate of the culture supernatant liquid flowing into the purification filter is changed by 50% or more at a set interval set to be 2 minutes to 30 minutes.

16. The method for producing an active pharmaceutical ingredient of a biopharmaceutical according to any one of claims 1 to 15,
wherein the culture supernatant liquid is allowed to flow into a sampling container connected downstream of the sterile filter in the coupling line.

17. The method for producing an active pharmaceutical ingredient of a biopharmaceutical according to claim 16,
wherein the culture supernatant liquid is allowed to flow into the sampling container using a supply pump provided in a branch line connecting the coupling line and the sampling container.

18. The method for producing an active pharmaceutical ingredient of a biopharmaceutical according to any one of claims 1 to 17,

    wherein the product is a protein.

19. The method for producing an active pharmaceutical ingredient of a biopharmaceutical according to any one of claims 1 to 18,

    wherein the cell is a cell derived from Chinese Hamster Ovary cell.

20. A system for producing a biopharmaceutical, the system comprising:

    a culture section where a culture step, in which cells are cultured in a culture liquid stored in a culture tank and the cells are removed from the culture liquid using a cell separation filter to obtain a culture supernatant liquid containing a product of the cells, is performed;
    a purification section where a purification step, in which the product is purified from the culture supernatant liquid to obtain an active pharmaceutical ingredient of a biopharmaceutical, is performed, the purification step including a purification filter of a single pass tangential flow filtration method;
    a coupling line coupling the culture section and the purification section; and
    a sterile filter which is provided in the coupling line, has a pore diameter larger than a pore diameter of the cell separation filter, and filters the culture supernatant liquid flowing from the culture section to the purification section through the coupling line.

21. An active pharmaceutical ingredient of a biopharmaceutical, which is produced by the method for producing an active pharmaceutical ingredient of a biopharmaceutical according to any one of claims 1 to 19.

# FIG. 1

A,D: CULTURE LIQUID   B,C: GAS CONTAINING AIR AND CARBON DIOXIDE
E: CONCENTRATED SOLUTION   F: GAS CONTAINING OXYGEN
G: CULTURE SUPERNATANT LIQUID

HAVING LARGER PORE DIAMETER THAN FILTER MEMBRANES OF CELL SEPARATION FILTER AND PURIFICATION FILTER
(0.1 $\mu$m OR MORE AND 1.0 $\mu$m OR LESS)

CULTURE SECTION

PURIFICATION SECTION

INTERMEDIATE SECTION

EP 4 501 335 A1

FIG. 2

EXCHANGE IN CASE WHERE INTEGRATED
FLOW RATE REACH SET AMOUNT

# FIG. 3

HAVING STRUCTURE IN WHICH FLAT FILTER PAPER MADE OF POLYETHERSULFONE IS FOLDED

44

CONSTANT FLOW RATE

41

99

57

59

100

MEASUREMENT RESULT

113

CONTROL DEVICE

13

112

111

110

60

58

SINGLE-USE BAG IN WHICH TWO RESIN-MADE SHEETS ARE LIQUID-TIGHTLY BONDED

29

$$0.001 \ \text{m}^2/\text{L} \leq (\text{FA/MCC}) \leq 0.01 \ \text{m}^2/\text{L}$$

MCC: MAXIMUM ACCOMMODATION CAPACITY OF CULTURE TANK

FA: FILTRATION AREA OF STERILE FILTER

101

105   104   102   106   108   109   61

103   107

CONSTANT FLOW RATE

INFLOW RATE < OUTFLOW RATE

CONSTANT FLOW RATE

EP 4 501 335 A1

# FIG. 4

# FIG. 5

13

122

CONTROL DEVICE

120

CULTURE SECTION

35 — DELIVERY PUMP

CONTROL SIGNAL

36 — FLOWMETER

MEASUREMENT RESULT

10

CPU

CULTURE SECTION CONTROL UNIT

135

STORAGE

CONTROL PROGRAM

130

INTERMEDIATE SECTION

43,50,70

62 — PINCH VALVE

CONTROL SIGNAL

FIRST SUPPLY PUMP

CONTROL SIGNAL

63 — FLOWMETER

MEASUREMENT RESULT

69

SECOND SUPPLY PUMP

CONTROL SIGNAL

ELECTRONIC SPRING SCALE

MEASUREMENT RESULT

113

11

INTERMEDIATE SECTION CONTROL UNIT

137

136

PURIFICATION SECTION

80 — SPTFF DEVICE

CONTROL SIGNAL

IMMUNAFFINITY CHROMATOGRAPHY DEVICE

CONTROL SIGNAL

81

12

PURIFICATION SECTION CONTROL UNIT

## FIG. 6

FLOW RATE

0.5 MC ≤ UL ≤ 1.0 MC
MC: MAXIMUM ACCOMMODATION CAPACITY OF INTERMEDIATE CONTAINER

0.1 MC ≤ LL < 0.5 MC
MC: MAXIMUM ACCOMMODATION CAPACITY OF INTERMEDIATE CONTAINER

(UL + LL) / 2

UL
PL
LL

OUTFLOW START
OUTFLOW STOP

TIME

FIRST SUPPLY PUMP — ON / OFF

PURIFICATION SECTION — ON / OFF

24

# FIG. 7

```
                        ┌─────────┐
                        │  START  │
                        └────┬────┘
                             │ ◄──────────────────────┐
ST100┐                       │                         │
  ┌──────────────────────────────────────────────┐    │
  │          FIRST SUPPLY PUMP STOPPED            │    │
  │ (OUTFLOW OF CULTURE SUPERNATANT LIQUID STOPPED),│  │
  │         PURIFICATION SECTION STOPPED          │    │
  └──────────────────────┬───────────────────────┘    │
ST110┐                   │                             │
  ╱────────────────────────────────────────╲  NO       │
 ⟨   FLOW RATE OF CULTURE SUPERNATANT       ⟩──────────┘
  ╲   LIQUID REACHED SET AMOUNT?            ╱
   ────────────────────┬─────────────────
                    YES │ ◄──────────────────────┐
ST120┐                  │                         │
  ┌──────────────────────────────────────────────┐    │
  │          FIRST SUPPLY PUMP OPERATED           │    │
  │ (OUTFLOW OF CULTURE SUPERNATANT LIQUID STARTED),│  │
  │        PURIFICATION SECTION OPERATED          │    │
  └──────────────────────┬───────────────────────┘    │
ST130┐                   │                             │
  ╱────────────────────────────────────────╲  NO       │
 ⟨   FLOW RATE OF CULTURE SUPERNATANT       ⟩──────────┘
  ╲ LIQUID REACHED LOWER LIMIT STORAGE AMOUNT?╱
   ────────────────────┬─────────────────
                    YES │
                  ┌─────────┐
                  │ RETURN  │
                  └─────────┘
```

# FIG. 8

## FIG. 9

## FIG. 10

# FIG. 11

# FIG. 12

# FIG. 13

EP 4 501 335 A1

# FIG. 14

150

151

41

58

HAVING STRUCTURE IN WHICH HOLLOW FIBERS MADE OF POLYETHERSULFONE ARE BUNDLED TOGETHER

## INTERNATIONAL SEARCH REPORT

| International application No. |
| --- |
| **PCT/JP2023/013429** |

### A. CLASSIFICATION OF SUBJECT MATTER

*A61K 35/12*(2015.01)i; *A61K 35/54*(2015.01)i; *C07K 1/00*(2006.01)i; *C07K 1/16*(2006.01)i; *C12M 3/00*(2006.01)i; *C12P 21/02*(2006.01)i; *C12P 21/08*(2006.01)i
FI:  A61K35/12; A61K35/54; C07K1/00; C07K1/16; C12M3/00 Z; C12P21/02 C; C12P21/08

According to International Patent Classification (IPC) or to both national classification and IPC

### B. FIELDS SEARCHED

Minimum documentation searched (classification system followed by classification symbols)

A61K35/12; A61K35/54; C07K1/00; C07K1/16; C12M3/00; C12P21/02; C12P21/08

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
| --- | --- | --- |
| X | JP 2016-510981 A (GENZYME CORP.) 14 April 2016 (2016-04-14) claims, paragraphs [0064]-[0068], [0126], [0127], fig. 1 | 21 |
| A | | 1-20 |
| X | JP 2017-522169 A (AMGEN INC.) 10 August 2017 (2017-08-10) paragraphs [0038], [0042], [0043], examples, fig. 1 | 21 |
| A | | 1-20 |
| A | JP 2018-518161 A (BAYER AKTIENGESELLSCHAFT) 12 July 2018 (2018-07-12) entire text | 1-21 |

☐ Further documents are listed in the continuation of Box C.　　☑ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **25 April 2023** | **16 May 2023** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

## INTERNATIONAL SEARCH REPORT
### Information on patent family members

| International application No. |
|---|
| **PCT/JP2023/013429** |

| Patent document cited in search report | | | Publication date (day/month/year) | Patent family member(s) | | | Publication date (day/month/year) |
|---|---|---|---|---|---|---|---|
| JP | 2016-510981 | A | 14 April 2016 | WO 2014/137903 A2<br>claims, p. 24, first paragraph to<br>p. 25, second paragraph, p. 51,<br>second paragraph to p. 52, first<br>paragraph, fig. 1 | | | |
| | | | | US 2014/0255994 | A1 | | |
| | | | | EP 2964663 | A2 | | |
| | | | | KR 10-2015-0125714 | A | | |
| | | | | CN 105377874 | A | | |
| JP | 2017-522169 | A | 10 August 2017 | WO 2015/175679 A2<br>paragraphs [0062], [0066],<br>[0067], examples, fig. 1 | | | |
| | | | | US 2017/0157566 | A1 | | |
| | | | | EP 3142775 | A2 | | |
| | | | | KR 10-2017-0004989 | A | | |
| | | | | CN 106794424 | A | | |
| JP | 2018-518161 | A | 12 July 2018 | WO 2016/177650 | A1 | | |
| | | | | US 2018/0135006 | A1 | | |
| | | | | EP 3015542 | A1 | | |
| | | | | KR 10-2018-0002783 | A | | |
| | | | | CN 107995924 | A | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2020033364 A

- JP 2018518161 A